# EUROPEAN PATENT SPECIFICATION

(11) **EP 2 105 099 B1**
(45) Date of publication and mention of the grant of the patent: **11.05.2011**
(21) Application number: 09004503.0
(22) Date of filing: 27.03.2009
(51) Int. Cl.: A61B 17/32, A61B 18/14, A61B 17/00

(54) **Ultrasonic operating apparatus**
Ultraschalloperationsvorrichtung
Appareil à fonctionnement à ultrasons

(30) Priority: 27.03.2008 US 56653
(43) Date of publication of application: 30.09.2009
(73) Proprietor: Olympus Medical Systems Corp., Tokyo 151-0072 (JP)
(72) Inventor: Hirai, Yuji, Hachioji-shi Tokyo 192-8512 (JP)
(74) Representative: von Hellfeld, Axel

(56) References cited:
- EP-A- 1 398 004
- US-A1- 2003 199 794
- US-A1- 2006 241 532

## Description

The present invention relates to an ultrasonic operating apparatus which can perform therapeutic treatment, such as incision, resection or coagulation, of a living body tissue by making use of composite energy of ultrasonic and high-frequency waves, and can also perform therapeutic treatment by high-frequency wave.

Jpn. Pat. Appln. KOKAI Publication No. 2003-265496 (patent document 1) and Jpn. Pat. Appln. KOKAI Publication No. 2005-278933 (patent document 2), for instance, disclose general examples of an ultrasonic operating apparatus which can perform therapeutic treatment, such as incision, resection or coagulation, of a living body tissue by making use of ultrasonic wave and can also perform therapeutic treatment by high-frequency wave.

Patent document 1 discloses a structure wherein a main body of an ultrasonic operating apparatus is assembled of three units, namely, a transducer unit, a probe unit and a handle unit. There is disclosed an example of means for selectively using a proper probe, which is suitable for a part to be subjected to therapeutic treatment or for a therapeutic method, by preventing erroneous combination of units when the three units, i.e. the transducer unit, probe unit and handle unit, are assembled. Specifically, when an improper sheath and a probe unit are combined, a contact point member is made impassable through an engaging section between the probe unit and the handle unit. Thereby, the units can be prevented from being assembled in erroneous combination.

Patent document 2 discloses a structure wherein a probe unit, a jaw unit, a sheath unit and a handle unit are assembled. A main body part, to which the jaw unit is attached, has an irregular shape which varies in accordance with model types. When the probe unit, jaw unit, sheath unit and handle unit are assembled, the jaw unit, which can be detachably attached to the handle unit that is suited to the probe unit, is attached. Thereby, erroneous combination can be prevented.

In the apparatus of patent document 1, the probe unit is made to have an irregular shape, thereby preventing erroneous combination. However, even if the probe unit is simply made to have an irregular shape, it is difficult to prevent erroneous combination when a combination between the handle unit and the transducer unit, which are suited to a part to be subjected to therapeutic treatment or a therapeutic method, is to be selected.

In the apparatus of patent document 2, erroneous combination at the main body part, to which the jaw unit is attached, is prevented by attaching the jaw unit that can be detachably attached to only the handle unit that is suited to the probe unit. Thus, in the case where the model type of the proper transducer unit is to be selected from a plurality of model types of transducer units, it is difficult to prevent erroneous combination between the transducer unit and the handle unit. US 2006/241532 discloses an ultrasonic apparatus according to the preamble of claim 1.

The present invention has been made in consideration of the above-described circumstance, and the object of the invention is to provide an ultrasonic operating apparatus which can prevent erroneous combination between an ultrasonic transducer unit and a handle unit, and can select a transducer unit which is suited to a part that is to be subjected to therapeutic treatment or a therapeutic method.

According to an aspect of the present invention, there is provided an ultrasonic operating apparatus comprising: an ultrasonic transducer which generates ultrasonic vibration; a probe unit which is coupled to the ultrasonic transducer and transmits the ultrasonic vibration from the ultrasonic transducer; an ultrasonic transducer unit having a casing section which stores the ultrasonic transducer; an electric cable which is provided at a proximal end portion of the ultrasonic transducer unit; a handle unit in which the probe unit is inserted and which includes a coupling end portion for coupling to a distal end portion of the ultrasonic transducer unit; and a coupling section which couples an outer periphery of the distal end portion of the ultrasonic transducer unit and a proximal end portion of the handle unit, wherein the coupling section includes a different-type assembly prevention section which prevents assembly between the ultrasonic transducer unit and the handle unit which are of different types.

Preferably, a first high-frequency electric path through which a high-frequency electric current is transmitted between the electric cable and the probe unit.

Preferably, a second high-frequency electric path through which a high-frequency electric current is transmitted between the electric cable and a sheath unit which contains the probe unit.

The different-type assembly prevention section includes an irregular-shaped section in which a shape of the coupling section between the distal end portion of the ultrasonic transducer unit and the proximal end portion of the handle unit is varied in accordance with a plurality of model types.

Preferably, the different-type assembly prevention section includes a key groove, which is disposed at different positions in accordance with a plurality of model types, and an engaging portion, which is engaged with the key groove, at the coupling section between the distal end portion of the ultrasonic transducer unit and the proximal end portion of the handle unit.

Preferably, the ultrasonic transducer unit is rotatable, relative to the handle unit, about an axis of the handle unit in a state of assembly in a normal position.

The invention can be more fully understood from the following detailed description when taken in conjunction with the accompanying drawings, in which:
FIG. 1 is a perspective view that schematically shows the entire structure of an ultrasonic operating apparatus which is a surgical operating apparatus according to a first embodiment of the present invention;
FIG. 2 is a perspective view showing a state in which coupling sections of the ultrasonic operating apparatus according to the first embodiment are disconnected;
FIG. 3A is a plan view showing a distal end portion of a sheath unit of the ultrasonic operating apparatus according to the first embodiment;
FIG. 3B is a plan view showing a distal end portion of a probe unit of the ultrasonic operating apparatus according to the first embodiment;
FIG. 4A is a longitudinal cross-sectional view showing a distal end portion of the sheath unit of the ultrasonic operating apparatus according to the first embodiment;
FIG. 4B is a longitudinal cross-sectional view showing an insulating coating of an inner peripheral surface of an inner cylinder;
FIG. 5 is a cross-sectional view taken along line V-V in FIG. 4A;
FIG. 6 is a cross-sectional view taken along line VI-VI in FIG. 4A;
FIG. 7 is a cross-sectional view taken along line VII-VII in FIG. 4A;
FIG. 8 is a longitudinal cross-sectional view showing a proximal end portion of the sheath unit of the ultrasonic operating apparatus according to the first embodiment;
FIG. 9A is a cross-sectional view taken along line IXA-IXA in FIG. 8;
FIG. 9B is a cross-sectional view taken along line IXB-IXB in FIG. 8;
FIG. 10 is a cross-sectional view taken along line X-X in FIG. 8;
FIG. 11 is a cross-sectional view taken along line XI-XI in FIG. 8;
FIG. 12 is a perspective view showing a connection tube body of the sheath unit of the ultrasonic operating apparatus according to the first embodiment;
FIG. 13 is a side view showing the connection tube body of the sheath unit of the ultrasonic operating apparatus according to the first embodiment;
FIG. 14 is a longitudinal cross-sectional view showing an internal structure of a handle unit of the ultrasonic operating apparatus according to the first embodiment;
FIG. 15A is a longitudinal cross-sectional view showing a state before engagement between the handle unit and the sheath unit at a cross-sectional position along line L15A-L15A in FIG. 14;
FIG. 15B is a longitudinal cross-sectional view showing a state after engagement between the handle unit and the sheath unit at the cross-sectional position along line L15A-L15A in FIG. 14;
FIG. 16 is a cross-sectional view taken along line L16-L16 in FIG. 14;
FIG. 17 is a cross-sectional view taken along line L17-L17 in FIG. 14;
FIG. 18 is a cross-sectional view taken along line L18-L18 in FIG. 14;
FIG. 19 is a cross-sectional view taken along line L19-L19 in FIG. 14;
FIG. 20 is a cross-sectional view taken along line L20-L20 in FIG. 14;
FIG. 21 is a cross-sectional view taken along line L21-L21 in FIG. 14;
FIG. 22 is a cross-sectional view taken along line L22-L22 in FIG. 14;
FIG. 23 is a cross-sectional view taken along line L23-L23 in FIG. 14;
FIG. 24 is a perspective view showing an electrode hold member of the ultrasonic operating apparatus according to the first embodiment;
FIG. 25 is a front view showing the electrode hold member of the ultrasonic operating apparatus according to the first embodiment;
FIG. 26 is a side view showing the electrode hold member of the ultrasonic operating apparatus according to the first embodiment;
FIG. 27 is a perspective view showing an electrode member of the ultrasonic operating apparatus according to the first embodiment;
FIG. 28 is a transverse cross-sectional view showing the electrode member of the ultrasonic operating apparatus according to the first embodiment;
FIG. 29 is a perspective view showing a state before the rotational engagement at the time when the handle unit and sheath unit of the ultrasonic operating apparatus according to the first embodiment are coupled;
FIG. 30 is a plan view showing a state before the rotational engagement at the time when the handle unit and sheath unit of the ultrasonic operating apparatus according to the first embodiment are coupled;
FIG. 31 is a perspective view showing a state after the rotational engagement at the time when the handle unit and sheath unit of the ultrasonic operating apparatus according to the first embodiment are coupled;
FIG. 32 is a plan view showing a state after the rotational engagement at the time when the handle unit and sheath unit of the ultrasonic operating apparatus according to the first embodiment are coupled;
FIG. 33 is a side view showing a state before an attachment member is assembled to a base member of a stationary handle of the handle unit of the ultrasonic operating apparatus according to the first embodiment;
FIG. 34 is a plan view showing a probe unit of the ultrasonic operating apparatus according to the first embodiment;
FIG. 35 is a cross-sectional view taken along line L35-L35 in FIG. 34;
FIG. 36 is a longitudinal cross-sectional view showing an internal structure of a front end portion of the transducer unit;
FIG. 37 is a side view showing a coupled state between the transducer unit and the handle unit;
FIG. 38A is a longitudinal cross-sectional view showing a proper combination state between a handle unit of a first model type and a transducer unit of the first model type according to the first embodiment;
FIG. 38B is a longitudinal cross-sectional view showing a proper combination state between a handle unit of a second model type and a transducer unit of the second model type according to the first embodiment;
FIG. 39A is a longitudinal cross-sectional view showing an erroneous combination state between the handle unit of the first model type and the transducer unit of the second model type according to the first embodiment;
FIG. 39B is a longitudinal cross-sectional view showing an erroneous combination state between the handle unit of the second model type and the transducer unit of the first model type according to the first embodiment;
FIG. 40A is a cross-sectional view taken along line L40A-L40A in FIG. 39A;
FIG. 40B is a cross-sectional view taken along line L40B-L40B in FIG. 39B;
FIG. 41 is a longitudinal cross-sectional view of a main part, which shows a proper combination state between a transducer unit and a handle unit according to a second embodiment of the present invention;
FIG. 42A is a cross-sectional view taken along line L42A-L42A in FIG. 41;
FIG. 42B is a longitudinal cross-sectional view for explaining an erroneous combination state between a handle unit of a first model type and a transducer unit of a second model type;
FIG. 43 is a transverse cross-sectional view of a main part, showing a modification according to the second embodiment;
FIG. 44 is a perspective view showing the shape of a C ring attachment of a first model type according to a third embodiment of the present invention; and
FIG. 45 is a perspective view showing the shape of a C ring attachment of a second model type according to the third embodiment.

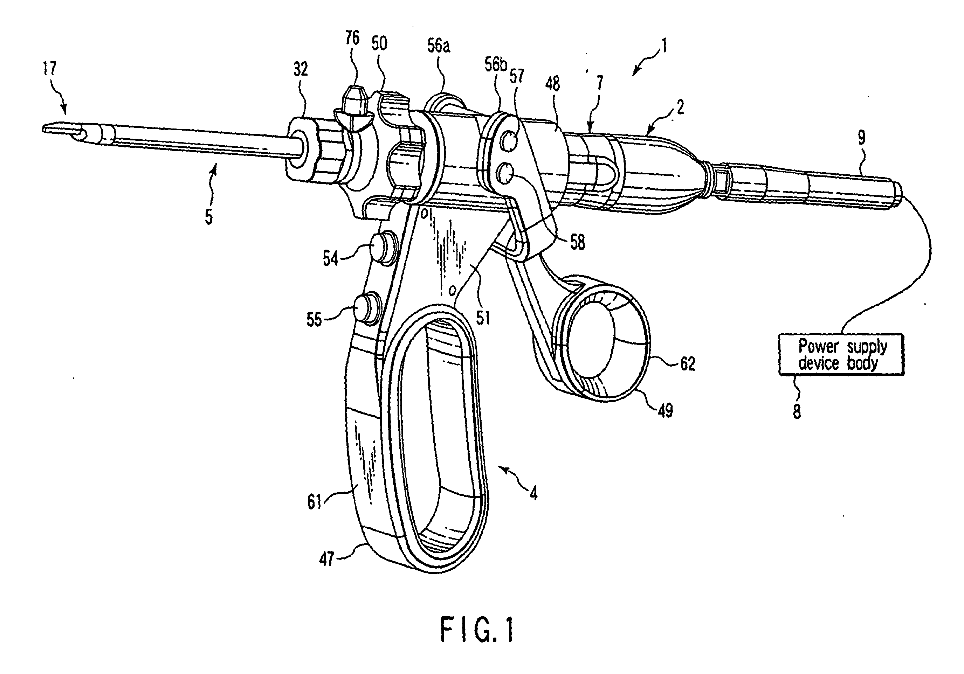

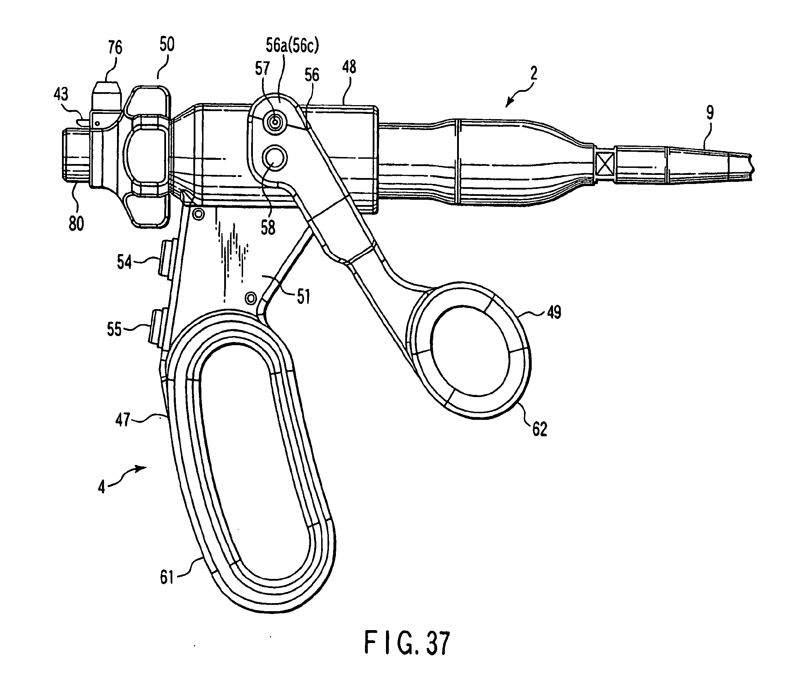

FIG. 1 to FIG. 40B show a first embodiment. FIG. 1 schematically shows the entire structure of a handpiece 1 of an ultrasonic operating apparatus according to the present embodiment. The ultrasonic operating apparatus of the present embodiment is an ultrasonic coagulation/incision operating apparatus which can perform therapeutic treatment, such as incision, resection or coagulation, of a living body tissue by making use of ultrasonic wave, and can also perform therapeutic treatment by high-frequency wave.

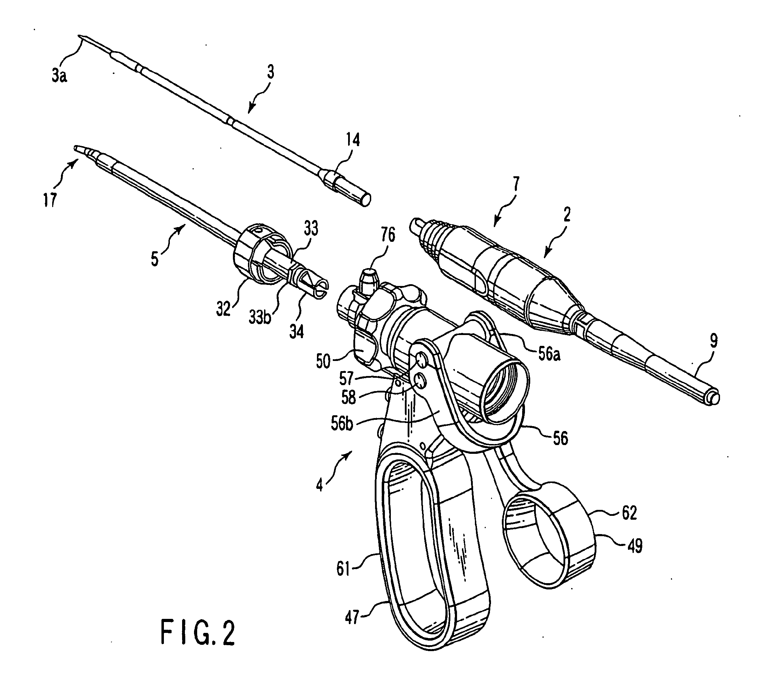

The handpiece 1, as shown in FIG. 2, comprises four units, namely, a transducer unit 2, a probe unit (probe section) 3, a handle unit (handle section) 4 and a sheath unit (sheath section) 5. These four units are detachably coupled.

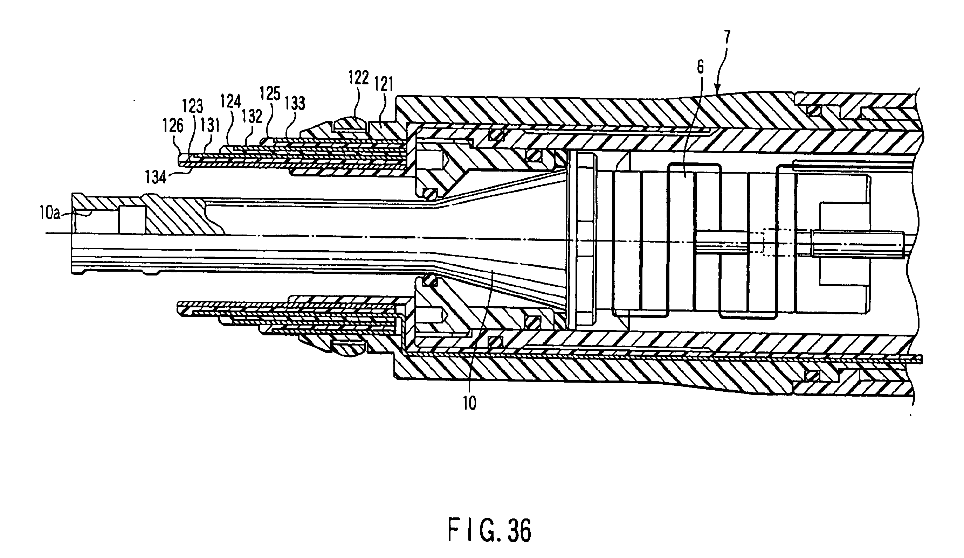

A transducer 6 (see FIG. 36), which will be described later, is assembled in the transducer unit 2. The transducer 6 generates ultrasonic vibration by a piezoelectric element which converts an electric current to ultrasonic vibration. An outside of the piezoelectric element is covered with a circular cylindrical transducer cover 7. Further, a cable 9 for supplying an electric current for generating ultrasonic vibration from a power supply device body 8 extends from a rear end of the transducer unit 2. A proximal end portion of a horn 10, which increases the amplitude of ultrasonic vibration, is coupled to a front end portion of the ultrasonic transducer 6 within the transducer cover 7. A screw hole portion 10a for attaching the probe is formed at a distal end portion of the horn 10.

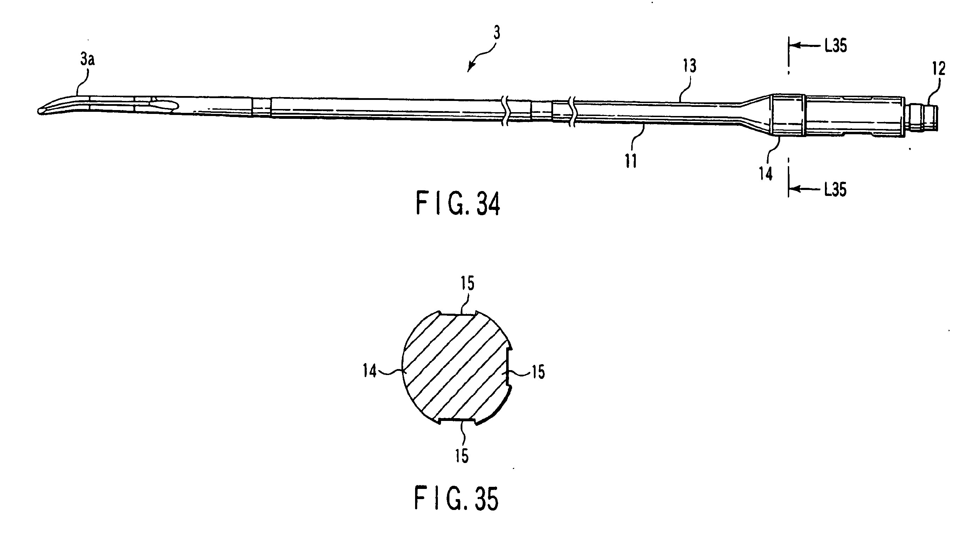

FIG. 34 shows the external appearance of the entire probe unit 3. The probe unit 3 is designed such that the entire length thereof may become an integer number of times of half-wave length of the ultrasonic vibration. The probe unit 3 includes a metallic rod-shaped vibration transmission member 11. A proximal end portion of the vibration transmission member 11 is provided with a screw portion 12 which is to be engaged with the screw hole portion 10a of the horn 10. The screw portion 12 is engaged with the screw hole portion 10a of the horn 10 of the transducer unit 2. Thereby, the probe unit 3 and the transducer unit 2 are assembled. At this time, a first high-frequency electric path 13, through which a high-frequency current is transmitted, is formed in the coupled body of the ultrasonic transducer 6 and the probe unit 3.

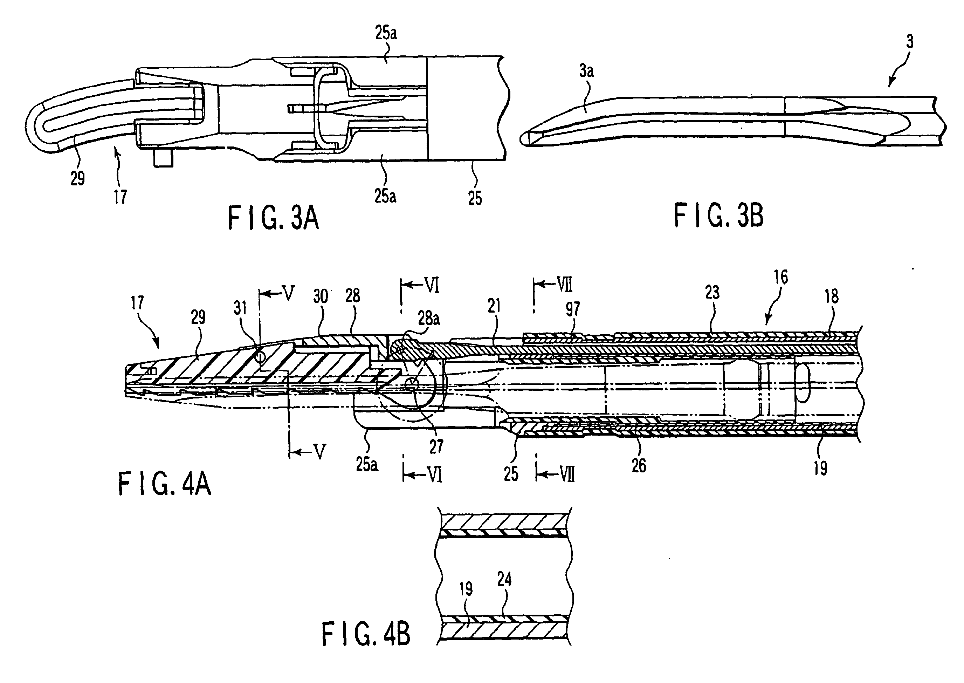

A probe distal end portion 3a, as shown in FIG. 3B, is provided at a distal end portion of the vibration transmission member 11. The probe distal end portion 3a is formed in a substantially J-shaped curved form. The cross-sectional area of the probe unit 3 is decreased in the axial direction at several nodes of vibration in the axial direction, so that an amplitude necessary for therapeutic treatment can be obtained at the probe distal end portion 3a. Rubber rings, which are formed of elastic material in an annular shape, are attached to several positions of nodes of vibration along the axial direction of the probe unit 3. These rubber rings prevent interference between the probe unit 3 and the sheath unit 5.

A flange portion 14 is provided at the position of the node of vibration on the most proximal end side in the axial direction of the probe unit 3. As shown in FIG. 35, engaging recess portions 15 each having a key groove shape are formed on the outer peripheral surface of the flange portion 14 at three positions in the circumferential direction thereof.

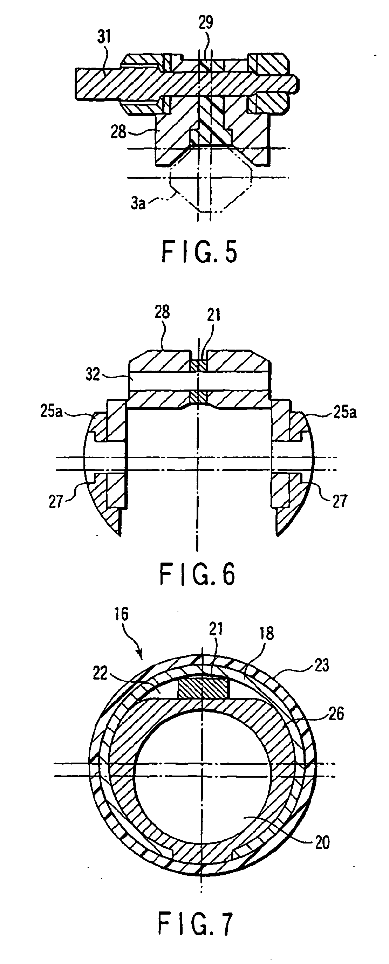

The sheath unit 5 includes a sheath body 16, which is formed of a circular cylindrical body, and a jaw 17 which is provided at a distal end of the sheath body 16. The sheath body 16, as shown in FIG. 7, includes a metallic outer cylinder 18 having a circular cross-sectional shape, and a metallic inner cylinder 19 having a non-circular cross-sectional shape, for example, a D-shaped cross section. A channel 22 for passing a driving shaft 21 of the jaw 17 is formed between the outer cylinder 18 and the inner cylinder 19.

As shown in FIG. 4A, the outer peripheral surface of the outer cylinder 18 is covered with an insulation tube 23. As shown in FIG. 4B, the inner peripheral surface of the inner cylinder 19 is covered with an insulation coating 24 of an insulating material. An insulation tube may be provided on the inner peripheral surface of the inner cylinder 19. Electrical insulation from the probe unit 3 is ensured by the insulation coating 24 on the inner cylinder 19. A proximal end portion of a substantially circular cylindrical distal end cover 25 is fixed to a distal end portion of the outer cylinder 18. A pipe-shaped hold member 26, which holds the probe unit 3 so as not to come in contact with the distal end cover 25, is attached to an inner peripheral surface of the proximal end portion of the distal end cover 25. A channel 20 having a circular cross section for passing the probe unit 3 is formed inside the hold member 26.

As shown in FIG. 3A, a pair of right and left jaw support portions 25a are formed at a distal end portion of the distal end cover 25 so as to extend toward the front direction of the outer cylinder 18. As shown in FIG. 6, a metallic jaw body 28 of the jaw 17 is rotatably attached to the jaw support portions 25a via two support pins 27.

The jaw 17, as shown in FIG. 3A, is formed in a substantially J-shaped curved form, which corresponds to the probe distal end portion 3a of the probe unit 3. The jaw 17 is configured to be opposed to the probe distal end 3a of the probe unit 3 and to be rotatable about the two support pins 27 (see FIG. 6). The jaw 17 is rotated and operated between an open position in which the jaw 17 is rotated in a direction away from the probe distal end 3a of the probe unit 3 and a closed position in which the jaw 17 is rotated in a direction toward the probe distal end 3a of the probe unit 3. By the operation of rotating the jaw 17 to its closed position, a living body tissue is held between the jaw 17 and the probe distal end 3a of the probe unit 3.

The jaw body 28 includes a hold member 29 which is formed of a resin such as PTFE, and a metallic hold portion attachment member 30 which holds the hold member 29. The hold member 29 is attached to the hold portion attachment member 30 by a pin 31 so as to be rotatable over a predetermined angle (see FIG. 5). Further, as shown in FIG. 4A, a distal end portion of the driving shaft 21 is coupled to the rear end of the jaw body 28 via a pin 28a. The driving shaft 21 extends in the inside of the distal end cover 25, and further extends between the outer cylinder 18 and inner cylinder 19 of the sheath body 16, as shown in FIG. 7, to the proximal end side of the sheath body 16.

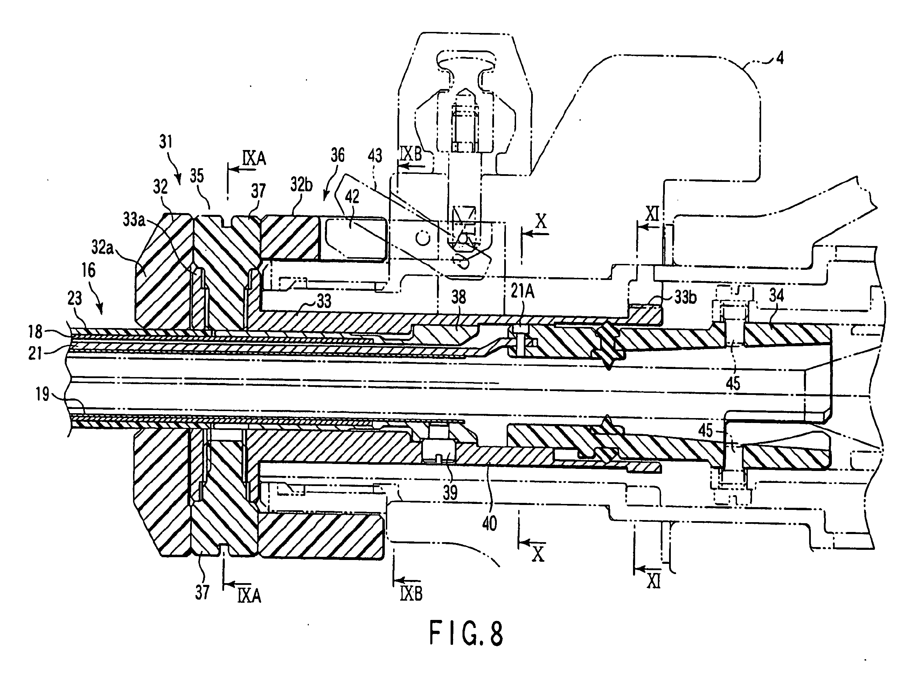

FIG. 8 shows a proximal end portion of the sheath body 16. The proximal end portion of the sheath body 16 is provided with an attachment/detachment mechanism section 31 for attachment/detachment to/from the handle unit 4. The attachment/detachment mechanism section 31 includes a circular cylindrical large-diameter knob member 32 which is formed of a resin material, a guide cylinder body 33 which is formed of a metallic circular cylinder body, and a circular cylindrical connection tube body 34 which is formed of a resin material.

The knob member 32 includes a ring-shaped first fixing portion 32a which is disposed at a front end part, and a circular cylindrical second fixing portion 32b which is disposed at a rear end part. The inner peripheral surface of the first fixing portion 32a is fixed to the outer peripheral surface of the proximal end portion of the sheath body 16. The second fixing portion 32b of the knob member 32 includes a fixing portion 35 of the guide cylinder body 33 that is disposed on the front end side, and an attachment/detachment portion 36 for attachment/detachment to/from the handle unit 4 that is disposed on the rear end part side.

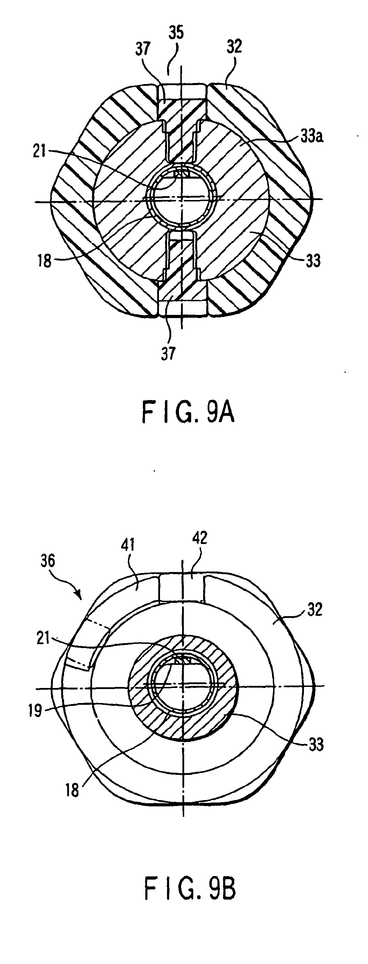

The guide cylinder body 33 includes a large-diameter front-end flange portion 33a which is disposed at the front end part, and an outer peripheral flange portion 33b which is disposed on the rear end part side. As shown in FIG. 9A, the front-end flange portion 33a of the guide cylinder body 33 is fixed to the knob member 32 by two resin-made fixing screws 37 in the state in which the front-end flange portion 33a is inserted in the knob member 32.

A metallic connection pipe 38 is provided inside the guide cylinder body 33. An inner peripheral surface of a front end portion of the connection pipe 38 is fixed by laser welding to the outer cylinder 18 of the sheath body 16. Further, the connection pipe 38 and guide cylinder body 33 are fixed by a metallic fixing screw 39. Thereby, the guide cylinder body 33, fixing screw 39, connection pipe 38, outer cylinder 18, distal end cover 25, support pins 27 and jaw body 28 are electrically connected, and a sheath-unit-side electric path 40 for transmission of a high-frequency electric current is formed.

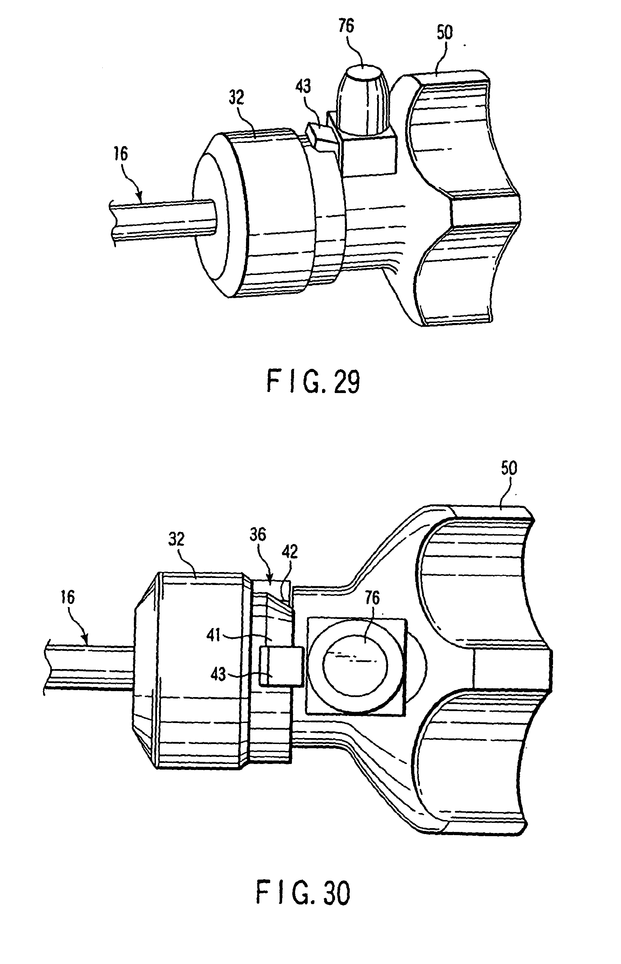

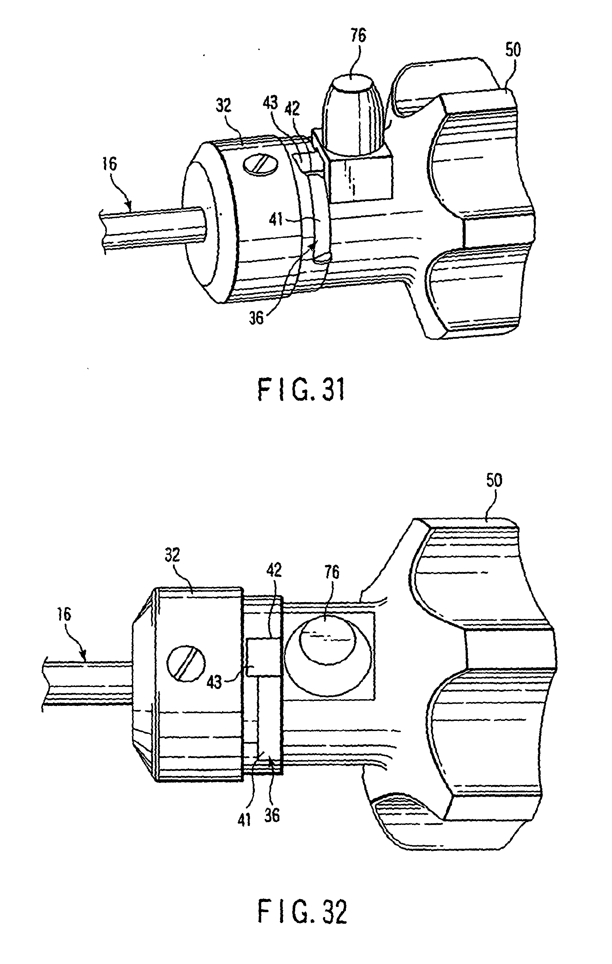

The attachment/detachment portion 36 of the knob member 32 includes an inclined-surface-shaped guide groove 41 which extends in the circumferential direction, as shown in FIG. 9B, and an engaging recess portion 42 which is formed at one end portion of the guide groove 41. The guide groove 41 has a tapered inclined surface having an outside diameter gradually decreasing toward the rear end portion side of the knob member 32. The engaging recess portion 42 is formed of a recess portion having a smaller diameter than the inclined surface of the guide groove 41. An engaging lever 43 (to be described later) on the handle unit 4 side is disengageably engaged in the engaging recess portion 42. FIG. 31 and FIG. 32 show a state in which the engaging lever 43 is engaged in the engaging recess portion 42, and FIG. 29 and FIG. 30 show a disengagement state in which the engaging lever 43 is disengaged from the engaging recess portion 42.

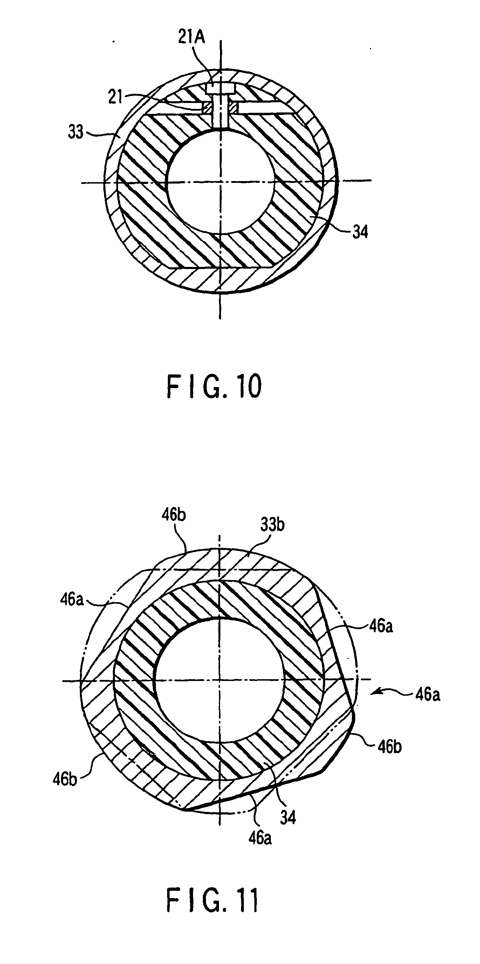

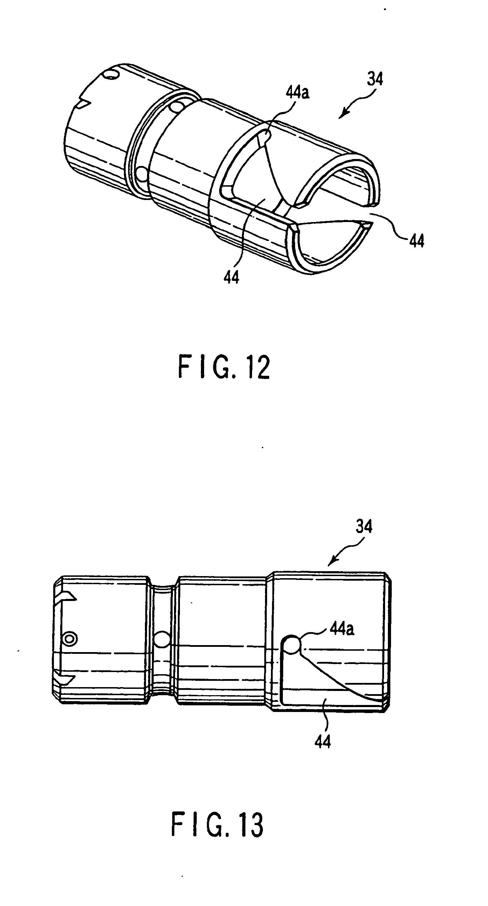

The connection tube body 34 is inserted in the guide cylinder body 33 so as to be slidable in the axial direction of the sheath body 16. A proximal end portion of the driving shaft 21 is fixed to a distal end portion of the connection tube body 34 via a pin 21A (see FIG. 10). A proximal end portion of the connection tube body 34 has two guide grooves 44 as shown in FIGS. 12 and 13. The guide grooves 44 are configured such that engaging pins 45 (to be described later) on the handle unit 4 side are disengageably engaged in the guide grooves 44, respectively. An engaging groove 44a, which restricts movement of the engaging pin 45 in the axial direction of the sheath body 16, is formed at a terminal end portion of the guide groove 44.

The outer peripheral flange portion 33b has a non-circular engaging portion 46. The engaging portion 46 has three cut-out flat-surface portions 46a at a plurality of locations on the circular outer peripheral surface of the outer peripheral flange portion 33b, for example, at three locations in this embodiment. Corner portions 46b, each having a greater diameter than the flat-surface portion 46a, are formed at connection parts between the three flat-surface portions 46a. Thereby, the engaging portion 46 with a substantially triangular cross section is formed on the outer peripheral flange portion 33b. It is not necessary that the non-circular engaging portion 46 have a substantially triangular shape. The non-circular engaging portion 46 may have any other non-circular shape, for instance, a polygon such as a rectangle or a pentagon.

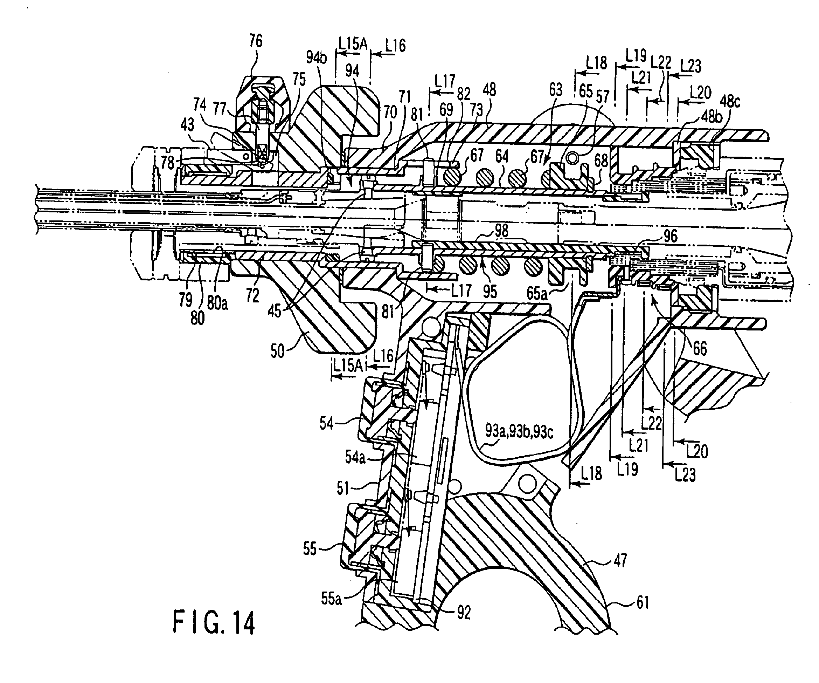

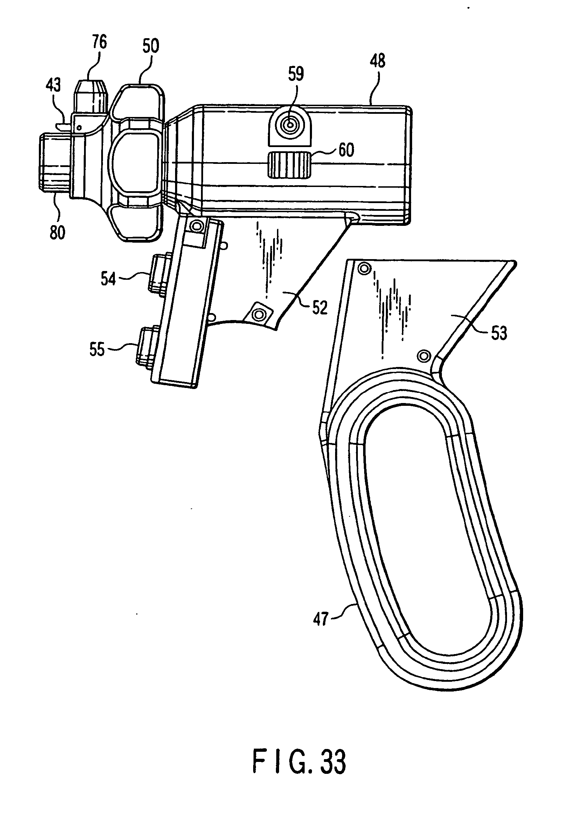

The handle unit 4 mainly includes a stationary handle 47, a hold cylinder 48, a movable handle 49, a rotational operation knob 50 and a handle-unit-side electric path 95 for transmission of a high-frequency electric current (see FIG. 14). The hold cylinder 48 is provided on the upper part of the stationary handle 47. A switch hold section 51 is provided between the stationary handle 47 and the hold cylinder 48. As shown in FIG. 33, the switch hold section 51 includes a switch attachment section 52 which is fixed to a lower end portion of the hold cylinder 48, and a cover member 53 which is fixed to an upper end portion of the stationary handle 47. The switch attachment section 52 has a plurality of hand switch buttons, for example, two hand switch buttons in this embodiment (e.g. a switch button 54 for incision and a switch button 55 for coagulation), which are push-button switches. As shown in FIG. 14, a switch 54a for incision, which is operated by the switch button 54 for incision, a switch 55a for coagulation, which is operated by the switch button 55 for coagulation, and a wiring circuit board 92 are assembled in the switch attachment section 52.
A wiring line 93a for incision, which has one end connected to the switch 54a for incision, a wiring line 93b for coagulation, which has one end connected to the switch 55a for coagulation, and a ground wiring line 93c, which has one end connected to a common terminal for grounding, are connected to the wiring circuit board 92. These three wiring lines 93a to 93c are looped and assembled in the switch hold section 51.

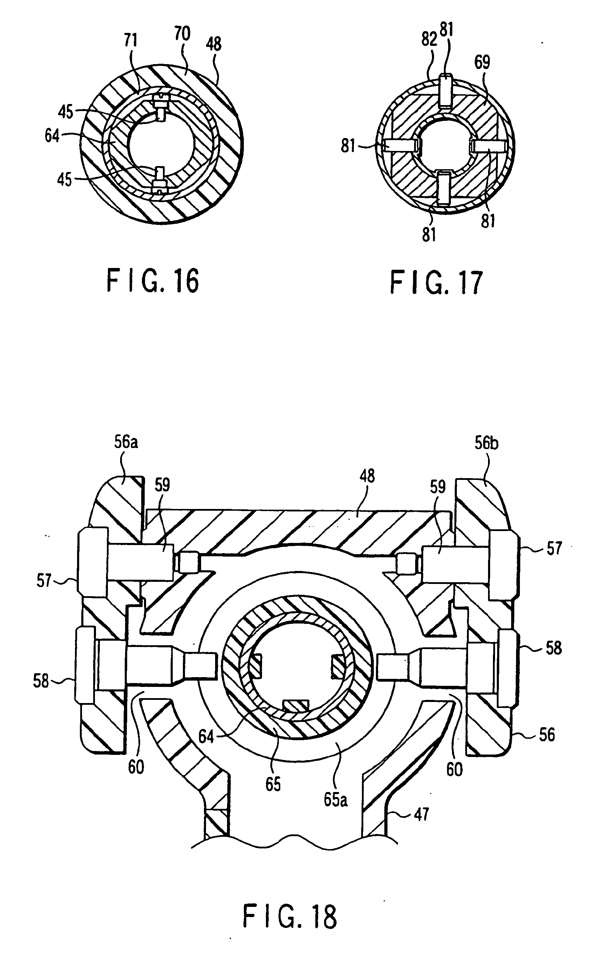

The movable handle 49 has a substantially U-shaped arm section 56 at an upper part thereof. The U-shaped arm section 56 includes two arms 56a and 56b, as shown in FIG. 18. The movable handle 49 is assembled to the hold cylinder 48 in the state in which the hold cylinder 48 is inserted between the two arms 56a and 56b.

Each of the arms 56a and 56b has a support pin 57 and an operation pin 58. A pin receiving hole portion 59 and a window portion 60 are formed in each of both side portions of the hold cylinder 48. The support pin 57 of each arm 56a, 56b is inserted in the pin receiving hole portion 59 of the hold cylinder 48. Thereby, an upper end portion of the movable handle 49 is rotatably supported on the hold cylinder 48 via the support pins 57.

Finger hook portions 61 and 62 are provided on lower end portions of the stationary handle 47 and movable handle 49, respectively. By hooking the fingers on the finger hook portions 61 and 62 and holding them, the movable handle 49 rotates via the support pins 57 and the movable handle 49 is opened/closed relative to the stationary handle 47.

The operation pins 58 of the movable handle 49 extend into the hold cylinder 48 through the window portions 60 of the hold cylinder 48. An operation force transmission mechanism 63, which transmits an operation force of the movable handle 49 to the driving shaft 21 of the jaw 17, is provided inside the hold cylinder 48. As shown in FIG. 14, the operation force transmission mechanism 63 mainly comprises a metallic circular cylindrical spring receiving member 64 and a resin-made slider member 65. The spring receiving member 64 is disposed coaxially with the center axis of the hold cylinder 48, and extends in the same direction as the direction of insertion of the probe unit 3.

A proximal end portion of the spring receiving member 64 is coupled to a circular cylindrical contact-point unit 66 (to be described later), which is fixed to a proximal end portion of the hold cylinder 48, so as to be rotatably about the axis and to be advancible/retreatable in the same direction as the direction of insertion of the probe unit 3. The above-described pair of engaging pins 45 on the handle unit 4 side are inwardly projectingly provided at a distal end portion of the spring receiving member 64. When the handle unit 4 and sheath unit 5 are coupled, the pair of engaging pins 45 on the handle unit 4 side are disengageably engaged with the engaging grooves 44a at the terminal end portion of the guide grooves 44 of the sheath unit 5.

A coil spring 67, the slider member 65, a stopper 68 and a spring receiver 69 are provided on an outer peripheral surface of the spring receiving member 64.
A front end portion of the coil spring 67 is fixed to the spring receiver 69. The stopper 68 restricts the position of movement of a rear end side of the slider member 65. The coil spring 67 is disposed between the spring receiver 69 and the slider member 65 with a fixed amount of mounting force.

An annular engaging groove 65a is formed along a circumferential direction in an outer peripheral surface of the slider member 65. As shown in FIG. 18, the operation pins 58 of the movable handle 49 are inserted and engaged in the engaging groove 65a. If the movable handle 49 is held and the movable handle 49 is closed relative to the stationary handle 47, the operation pins 58 rotate about the support pins 57 in accordance with the rotational operation of the movable handle 49 at this time. The slider member 65, which is in interlock with the rotation of the support pins 57, moves forward in the axial direction. At this time, the spring receiving member 64, which is coupled to the slider member 65 via the coil spring 67, moves forward/backward together with the slider member 65. Thereby, the operation force of the movable handle 49 is transmitted to the connection tube body 34 via the pair of engaging pins 45, and the driving shaft 21 of the jaw 17 moves forward. Thus, the jaw body 20 of the jaw 17 rotates via the support pin 21.

Further, when a living body tissue is clamped between the hold member 29 of the jaw 17 and the probe distal end portion 3a of the probe unit 3 by this operation, the hold member 29 rotates over a certain angle about the pin 31 in accordance with the bending of the probe distal end portion 3a so that force uniformly acts over the entire length of the hold member 29. In this state, ultrasonic wave is output and a living body tissue, such as a blood vessel, can be coagulated or incised.

An annular bearing portion 70 is formed at a front end portion of the hold cylinder 48. The bearing portion 70 is metallic, and a circular cylindrical rotation transmission member 71 is coupled to the bearing portion 70 so as to be rotatable about the axis. The rotation transmission member 71 includes a projecting portion 72 which projects forward of the bearing portion 70, and a large-diameter portion 73 which extends to the inner side of the hold cylinder 48 from the bearing portion 70.

The rotational operation knob 50 is fitted and fixed on the projecting portion 72. The engaging lever 43 is provided at the front end portion of the rotational operation knob 50. An intermediate portion of the engaging lever 43 is rotatably coupled to the projecting portion 72 via a pin 74. A proximal end portion of the engaging lever 43 extends to the inside of a lever receiving recess portion 75 which is formed in a front surface of the rotational operation knob 50. An operation button 76 for operating the engaging lever 43 in such a direction as to disengage the engaging lever 43 is provided on an outer peripheral surface of the front end portion of the rotational operation knob 50. An operation pin 77, which is disposed downward, is provided so as to project from the operation button 76. The operation pin 77 extends to the inside of the lever receiving recess portion 75 through a wall hole of the rotational operation knob 50. A proximal end portion of the engaging lever 43 is rotatably coupled to a lower end portion of the operation pin 77 via a pin 78.

A removal prevention ring 80 for the rotational operation knob 50 is provided on a distal end portion of the projecting portion 72. A male threaded portion 79 is formed on the distal end portion of the projecting portion 72. A female threaded portion 80a, which is to be meshed with the male threaded portion 79, is formed on an inner peripheral surface of the removal prevention ring 80. The female threaded portion 80a of the removal prevention ring 80 is meshed and engaged with the male threaded portion 79 of the projecting portion 72, and thereby the rotational operation knob 50 is fixed to the rotation transmission member 71.

As shown in FIG. 17, the spring receiver 69 of the spring receiving member 64 is provided with four metallic positioning pins 81 which project radially outward. An elongated engaging hole portion 82, in which one pin 81 of the spring receiving member 64 is inserted, is formed in the large-diameter portion 73 of the rotation transmission member 71. The engaging hole portion 82 extends in the same direction as the direction of insertion of the probe unit 3. Thereby, when the movable handle 49 is operated, the pin 81 is moved along the engaging hole portion 82 and thus the advancing/retreating movement of the spring receiving member 64 is prevented from being transmitted to the rotation transmission member 71.

On the other hand, when the rotational operation knob 50 is rotated, the rotational movement of the rotation transmission member 71, which rotates together with the rotational operation knob 50, is transmitted to the spring receiving member 64 via the pin 81. Thereby, when the rotational operation knob 50 is rotated, the assembly unit of the rotation transmission member 71, pin 81, spring receiving member 64, slider member 65 and coil spring 67 within the hold cylinder 48 is rotated together with the rotational operation knob 50 as one body about the axis thereof.

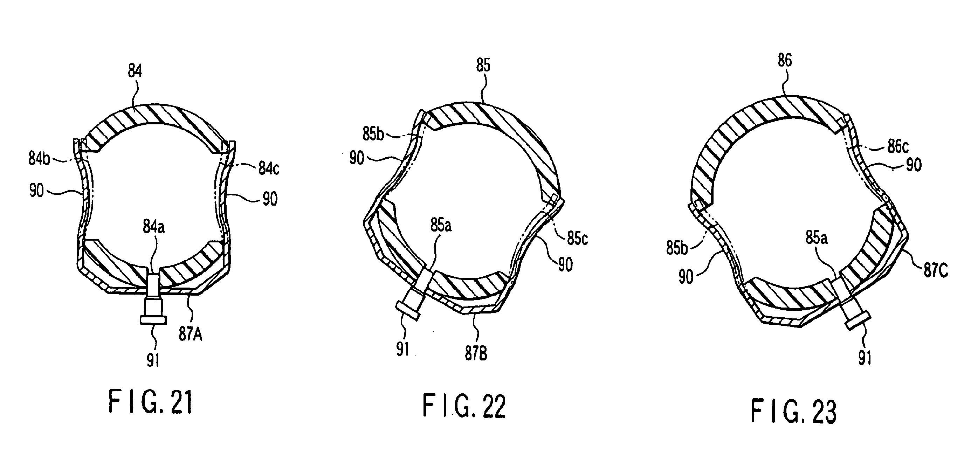

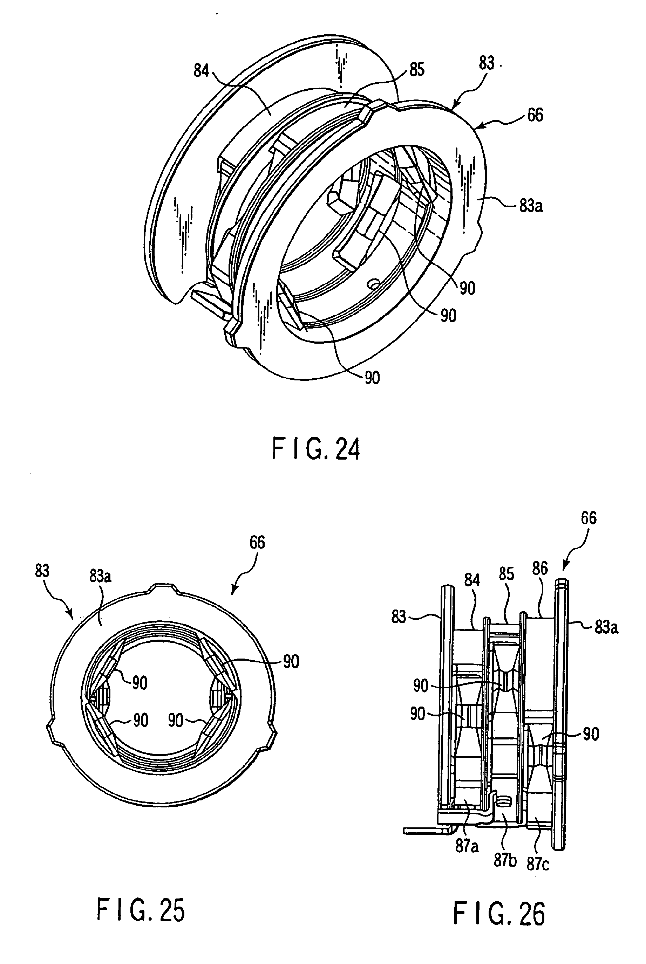

FIGS. 24 to 26 show the circular cylindrical contact-point unit 66. The contact-point unit 66 includes a circular cylindrical electrode hold member 83 which is formed of a resin. As shown in FIG. 26, the electrode hold member 83 includes three (first to third) electrode receiving sections 84, 85 and 86 with different outside diameters. The first electrode receiving section 84 on the distal end side has a smallest diameter, and the third electrode receiving section 86 on the rear end side has a greatest diameter. As shown in FIG. 21, the first electrode receiving section 84 has one contact-point member fixing hole 84a, and two through-holes 84b and 84c. A center line of the two through-holes 84b and 84c is set to be perpendicular to a center line of the contact-point member fixing hole 84a. Similarly, as shown in FIG. 22, the second electrode receiving section 85 has one contact-point member fixing hole 85a, and two through-holes 85b and 85c. As shown in FIG. 23, the third electrode receiving section 86 has one contact-point member fixing hole 86a, and two through-holes 86b and 86c.

The positions of the contact-point member fixing hole 85a of the first electrode receiving section 84, the contact-point member fixing hole 86a of the second electrode receiving section 85 and the contact-point member fixing hole 86a of the third electrode receiving section 86 are displaced in the circumferential direction of the electrode hold member 83.

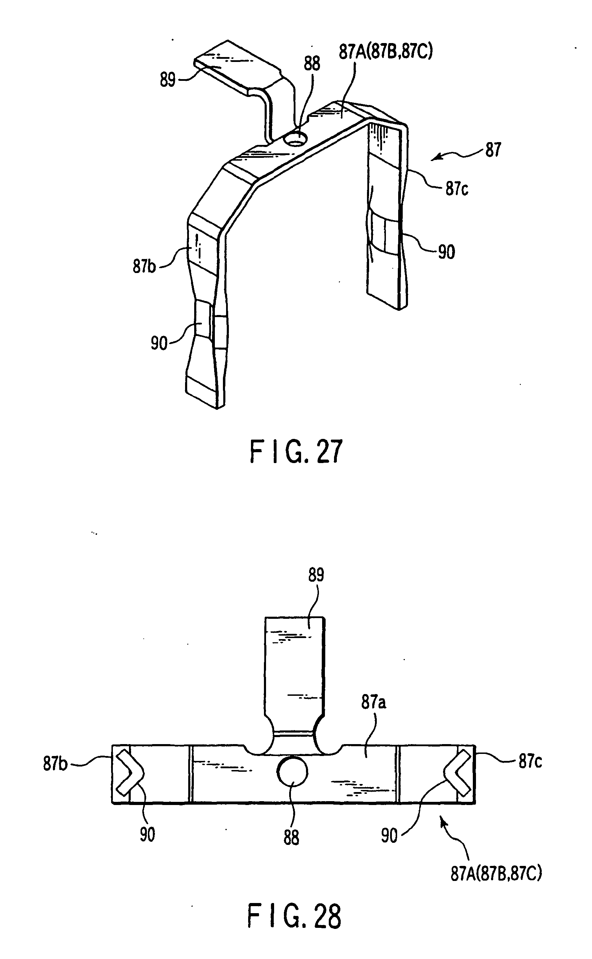

FIG. 27 and FIG. 28 show electrode members 87A, 87B and 87C which are assembled to the first to third electrode receiving sections 84, 85 and 86. These electrode members 87A, 87B and 87C are formed in the same shape. In the description below, only the electrode member 87A, which is assembled to the first electrode receiving section 84, is described. The common parts of the electrode members 87B and 87C of the other second and third electrode receiving sections 85 and 86 are denoted by like reference numerals, and a description thereof is omitted.

The electrode member 87A includes one straight stationary portion 87a and two bend portions 87b and 87c. One bend portion 87b is disposed at one end of the straight stationary portion 87a, and the other bend portion 87c is disposed at the other end of the straight stationary portion 87a. Thereby, as shown in FIG. 27, the electrode member 87A is formed and bent in a substantially U shape.

A hole 88 and an L-shaped wiring connection portion 89 are provided at a central position of the stationary portion 87a. Inwardly curved waist portions 90 are formed at central positions of the two bend portions 87b and 87c.

When the electrode member 87A is assembled to the first electrode receiving section 84, a fixing pin 91 is inserted in the hole 88 of the stationary portion 87a of the electrode member 87A and in the contact-point member fixing hole 85a of the first electrode receiving section 84. The electrode member 87A is fixed to the first electrode receiving section 84 by the fixing pin 91. At this time, the waist portion 90 of one bend portion 87b of the electrode member 87A is disposed in one through-hole 85b of the first electrode receiving section 84, and the waist portion 90 of the other bend portion 87c of the electrode member 87A is disposed in the other through-hole 85c. The same applies when the electrode member 87B is assembled to the second electrode receiving section 85 and the electrode member 87C is assembled to the third electrode receiving section 86.

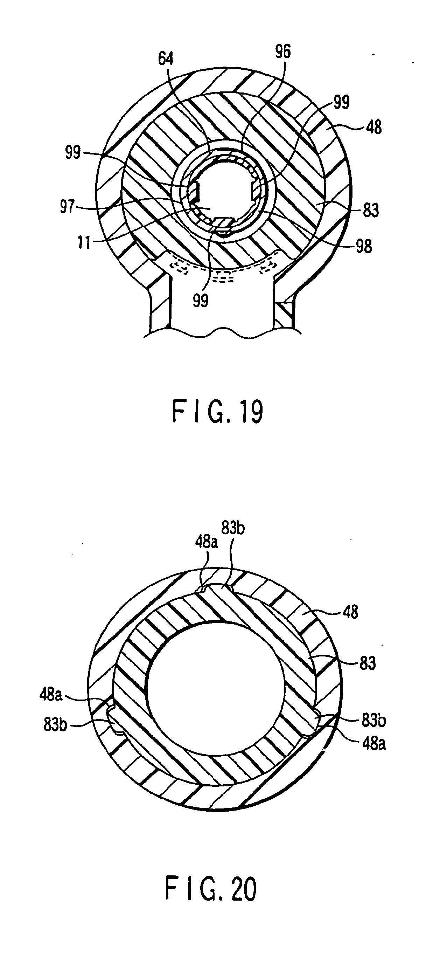

As shown in FIG. 27, a large-diameter fixing flange portion 83a is formed at a rear end portion of the electrode hold member 83 of the contact-point unit 66. As shown in FIG. 20, engaging projection portions 83b are projectingly provided on the outer peripheral surface of the fixing flange portion 83a at a plurality of locations, for example, at three locations in this embodiment. Engaging recess portions 48a are formed in an inner peripheral surface of the rear end portion of the hold cylinder 48 at positions corresponding to the three engaging projection portions 83b of the fixing flange portion 83a. In the case where the electrode hold member 83 is assembled in the hold cylinder 48, the three engaging projection portions 83b of the fixing flange portion 83a are inserted, engaged and fixed in the engaging recess portions 48a of the hold cylinder 48. Thereby, the rotation of the electrode hold member 83 about the axis thereof, relative to the hold cylinder 48, is restricted.

A stepped portion 43b, which comes in contact with the fixing flange portion 83a of the electrode hold member 83, is formed on the hold cylinder 48. The electrode hold member 83 is fixed to the hold cylinder 48 by a fixing screw 48c in the state in which the fixing flange portion 83a of the electrode hold member 83 abuts upon the stepped portion 43b (see FIG. 14). Thereby, the axial movement of the electrode hold member 83, relative to the hold cylinder 48, is restricted.

End portions of three wiring lines 93a to 93c, which are assembled in the switch hold section 51, are connected to the wiring connection portions 89 of the three electrode members 87A, 87B and 87C that are assembled to the contact-point unit 66.

Further, as shown in FIG. 19, the contact-point unit 66 is provided with a substantially C-shaped electric contact-point member 96 which is formed of a metallic plate spring. The electric contact-point member 96 is connected to the outer-peripheral surface of the proximal end portion.of the spring receiving member 64.

The handle-unit-side electric path 95 is composed of the electric contact-point member 96, spring receiving member 64, positioning pin 81 and rotation transmission member 71.

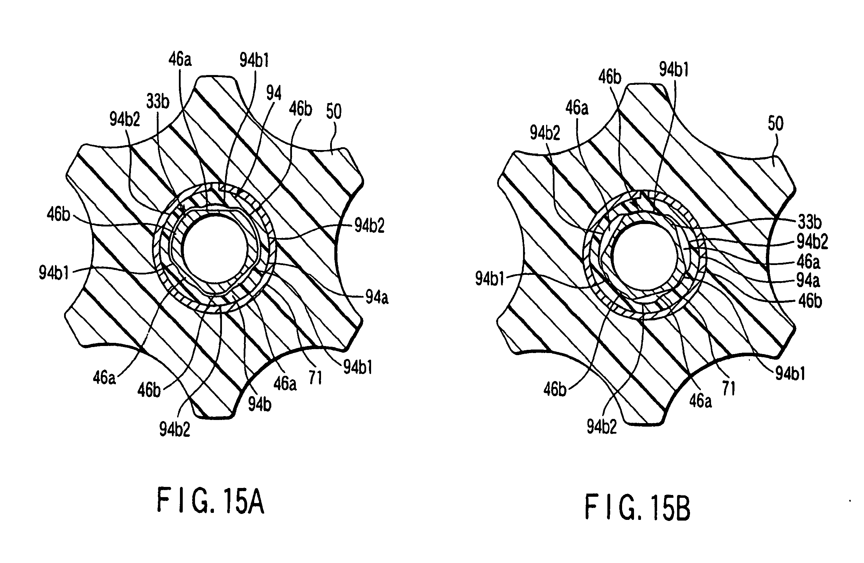

Engaging means 94, which is disengageably engaged with the outer peripheral flange portion 33b of the sheath unit 5, is provided on the inner peripheral surface of the rotation transmission member 71 at a substantially central position along the axial direction. As shown in FIGS. 15A and 15B, the engaging means 94 includes an insertion hole portion 94a in which the outer peripheral flange portion 33b is inserted when the sheath unit 5 and handle unit 4 are coupled, and an electrically conductive rubber ring (urging means) 94b which is disposed within the insertion hole portion 94a.

The shape of the inner peripheral surface of the electrically conductive rubber ring 94b is substantially the same as the shape of the engaging portion 46 of the outer peripheral flange portion 33b. Specifically, the inner peripheral surface of the electrically conductive rubber ring 94b has three cut-out flat-surface portions 94b1 at a plurality of locations on the circular inner peripheral surface, for example, at three locations in this embodiment, and three corner portions 94b2 which are located at connection parts between the three flat-surface portions 94b1 and have greater diameters than the flat-surface portions 94b1. Thereby, the electrically conductive rubber ring 94b has a substantially triangular cross-sectional shape. Thus, as shown in FIG. 15A, the electrically conductive rubber ring 94b is held in a natural, non-compressed position in the positional state in which the inner peripheral surface shape of the electrically conductive rubber ring 94b corresponds to the engaging portion 46 of the outer peripheral flange portion 33b, that is, in the state in which the three corner portions 46b of the outer peripheral flange portion 33b correspond in position to the three corner portions 94b2 of the electrically conductive rubber ring 94b. On the other hand, by rotating the handle unit 4 and the sheath unit 5 relative to each other about the center axis of the sheath unit 5, the position of the electrically conductive rubber ring 94b is switched to a pressure contact'position, as shown in FIG. 15B, where the electrically conductive rubber ring 94b is pressed on the three corner portions 46b of the outer peripheral flange portion 33b. At this time, the three corner portions 46b of the outer peripheral flange portion 33b are put in contact with, and pressed by, the three flat-surface portions 94b1 of the electrically conductive rubber ring 94b.

In the present embodiment, at the time of coupling the sheath unit 5 and handle unit 4, when the outer peripheral flange portion 33b of the sheath unit 5 is inserted straight into the electrically conductive rubber ring 94b (see FIG. 29 and FIG. 30), the electrically rubber ring 94b is held in the natural, non-compressed position, as shown in FIG. 15A. At this time, the engaging lever 43 on the handle unit 4 side is held in the state in which the engaging lever 43 rests on the inclined surface of the guide groove 41 of the handle member 32 of the sheath unit 5. Subsequently, the handle member 32 of the sheath unit 5 is rotated about the axis, relative to the handle unit 4. Thereby, as shown in FIG. 31 and FIG. 32, the engaging lever 43 on the handle unit 4 side is inserted and engaged in the engaging recess portion 42 at one end portion of the guide groove 41. At this time, as shown in FIG. 15B, the electrically conductive rubber ring 94b is switched to the pressure contact position where the electrically conductive rubber ring 94b is put in pressure contact with the three corner portions 46b of the outer peripheral flange portion 33b. Thereby, a sheath-unit-side electric path 40 (formed between the guide cylindrical body 33, fixing screw 39, coupling pipe 38, outer cylinder 18, distal end cover 25, support pin 27 and jaw body 28) and a handle-unit-side electric path 95 (formed between the electric contact-point member 96, spring receiving member 64, positioning pin 81 and rotation transmission member 71) are electrically connected via the electrically conductive rubber ring 94b. In this case, a second high-frequency electric path 97, which transmits a high-frequency current, is formed in the coupled body of the sheath unit 5 and handle unit 4.

As shown in FIG. 19, the handle unit 4 includes a tubular member 98 which is formed of an insulating material on the inner peripheral surface of the spring receiving member 64. The tubular member 98 is fixed on the inner peripheral surface of the spring receiving member 64. Thereby, when the probe unit 3 and the handle unit 4 are connected, the first high-frequency electric path 13 and the second high-frequency electric path 97 are insulated by the tubular member 98. An inner peripheral surface of the tubular member 98 has three engaging projection portions 99 which correspond to the three engaging recess portions 15 (see FIG. 35) of the flange portion 14 of the probe unit 3. When the probe unit 3 and handle unit 4 are connected, the three engaging projection portions 99 of the tubular member 98 are disengageably engaged with the three engaging recess portions 15 of the flange portion 14 of the probe unit 3. Thereby, the rotational-directional position between the probe unit 3 and the tubular member 98 of the handle unit 4 is restricted. Hence, when the rotational operation knob 50 is rotated, the coupled body of the probe unit 3 and transducer unit 2 is rotated as one body together with the assembly unit within the hold cylinder 48.

The engaging section between the flange portion 14 of the probe unit 3 and the tubular member 98 is not limited to the above-described structure. For example, the tubular member 98 may be formed to have a D-shaped cross section, and the flange portion 14 of the probe unit 3 may be formed to have a corresponding D-shaped cross section.

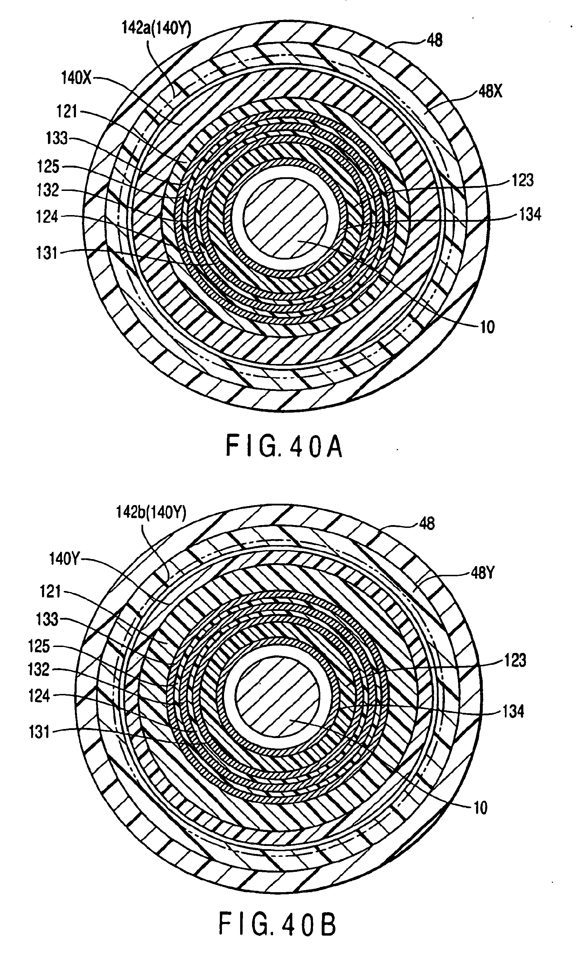

A front end portion of the transducer unit 2 is detachably coupled to the contact-point unit 66. As shown in FIG. 40, two wiring lines 101 and 102 for the ultrasonic transducer, two wiring lines 103 and 104 for transmission of high-frequency electricity and three wiring lines 105, 106 and 107, which are connected to a wiring circuit board 92 within the switch hold section 51, are assembled in the single cable 9 at the rear end of the transducer unit 2. Distal end portions of the two wiring lines 101 and 102 for the ultrasonic transducer are connected to the ultrasonic transducer 6. A distal.end portion of one wiring line 103 for transmission of high-frequency electricity is connected to the ultrasonic transducer 6.

First to fourth electrically conductive plates 111 to 114 for electric connection are provided at the rear end of the transducer unit 2. A distal end portion of the other wiring line 104 for transmission of high-frequency electricity is connected to the first conductive plate 111. The three wiring lines 105, 106 and 107 are connected to the second to fourth conductive plates 112 to 114.

FIG. 36 shows the internal structure of a front end portion of the transducer unit 2. A connection cylindrical portion 121 is formed at the distal end portion of the transducer cover 7. A C-ring 122 having a partly cut-out plate-spring shape is mounted on the outer peripheral surface of the connection cylindrical body 121. Three (first to third) cylindrical portions 123 to 125 with different outside diameters are projectingly provided on the inside of the connection cylindrical portion 121. The first cylindrical portion 123 has a smallest outside diameter and has a greatest length of projection from the distal end of the connection cylindrical body 121. The second cylindrical portion 124 has an outside diameter, which is greater than the outside diameter of the first cylindrical portion 123, and has a length of projection from the distal end of the connection cylindrical body 121, which is less than the length of projection of the first cylindrical portion 123. The third cylindrical portion 125 has a greatest outside diameter and has a length of projection from the distal end of the connection cylindrical body 121, which is less than the length of projection of the second cylindrical portion 124. A first cylindrical contact-point member 131 is mounted on the outer peripheral surface of the first cylindrical portion 123. Similarly, a second cylindrical contact-point member 132 is mounted on the outer peripheral surface of the second cylindrical portion 124, and a third cylindrical contact-point member 133 is mounted on the outer peripheral surface of the third cylindrical portion 125. The second conductive plate 112 is connected to the first contact-point member 131, the third conductive plate 113 is connected to the second contact-point member 132, and the fourth conductive plate 114 is connected to the third contact-point member 133.

A fourth contact-point member 134 having a circular cylindrical shape is mounted on the inner peripheral surface of the first cylindrical portion 123. The fourth contact-point member 134 is connected to the first conductive plate 111.

When the handle unit 4 and the transducer unit 2 are coupled, the contact-point unit 66 of the handle unit 4 and the front end portion of the transducer unit 2 are connected. At this time, the electrode member 87A of the contact-point unit 66 and the first contact-point member 131 of the transducer unit 2 are connected. At the same time, the electrode member 87B of the contact-point unit 66 and the second contact-point member 132 of the transducer unit 2 are connected, the electrode member 87C of the contact-point unit 66 and the third contact-point member 133 of the transducer unit 2 are connected, and the C-shaped electric contact-point member 96 of the contact-point unit 66 and the fourth contact-point member 134 of the transducer unit 2 are connected.

The handpiece 1 of the present embodiment is provided with an erroneous combination prevention section (different-type assembly prevention section) which prevents, when the transducer unit 2 and the handle unit 4 are to be coupled, coupling of an erroneous combination of units, other than a combination of matching units. This erroneous combination prevention section is configured such that a C ring attachment 140 which holds the C ring 122 of the transducer unit 2, and the hold cylinder 48 of the handle unit 4 are designed to have different dimensions and shapes in accordance with model types.

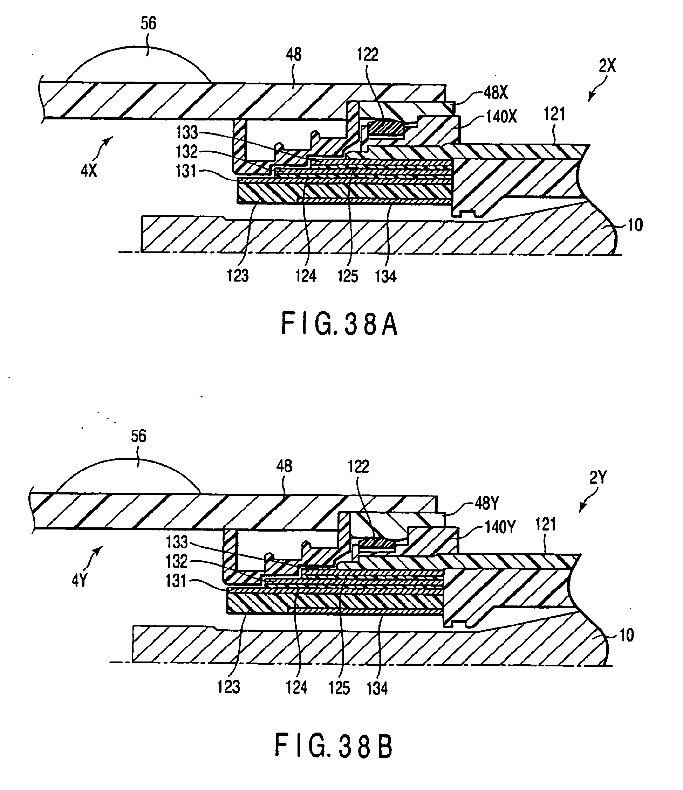

FIG. 38A is a longitudinal cross-sectional view showing a proper combination state between a handle unit 4X of a first model type and a transducer unit 2X of the first model type in the present embodiment. The hold cylinder of the handle unit 4X of the first model type is 48X, and the C ring attachment of the transducer unit 2X is 140X.

FIG. 38B is a longitudinal cross-sectional view showing a proper combination state between a handle unit 4Y of a second model type and a transducer unit 2Y of the second model type in the first embodiment. The hold cylinder of the handle unit 4Y of the second model type is 48Y, and the C ring attachment of the transducer unit 2Y is 140Y.

In the erroneous combination prevention section of the present embodiment, the hold cylinder 48X of the handle unit 4X of the first model type and the hold cylinder 48Y of the handle unit 4Y of the second model type are designed to have different dimensions and shapes. Similarly, the C ring attachment 140X of the transducer unit 2X of the first model type and the C ring attachment 140Y of the transducer unit 2Y of the second model type are designed to have different dimensions and shapes.

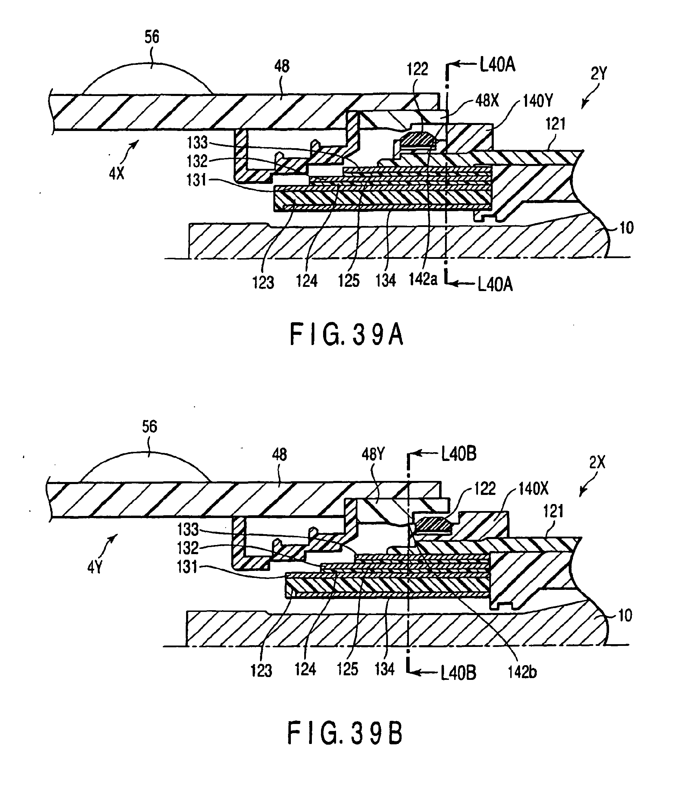

FIG. 39A is a longitudinal cross-sectional view showing an erroneous combination state between the handle unit 4X of the first model type and the transducer unit 2Y of the second model type. Similarly, FIG. 39B is a longitudinal cross-sectional view showing an erroneous combination state between the handle unit 4Y of the second model type and the transducer unit 2Y of the first model type. As shown in FIG. 39A and FIG. 39B, if a transducer unit 2 and a handle unit 4, which do not match with each other, are erroneously combined to be coupled, these units cannot be coupled to the proper position. Thus, only in the case of a proper combination of the handle unit 4 and transducer unit 2 (i.e. only in the case of a combination of the units of the same model type), the handle unit 4 and transducer unit 2 can be combined.

Next, the operation of the present embodiment is described. The handpiece 1 of the ultrasonic operating apparatus of the present embodiment, as shown in FIG. 2, comprises four units, namely, the transducer unit 2, probe unit 3, handle unit 4 and sheath unit 5, which are detachable. When the handpiece 1 is used, the transducer unit 2 and the probe unit 3 are coupled. Then, the handle unit 4 and the sheath unit 5 are coupled.

At the time of performing the work for coupling the transducer unit 2 and probe unit 3 of the handpiece 1 of this embodiment, a plurality of types, e.g. two types in the present embodiment, of handle units 4 (handle unit 4X of the first model type or transducer unit 2X of the first model type) and two types of transducer units 2 (handle unit 4Y of the second model type or transducer unit 2Y of the second model type) are selected in accordance with the purposes of therapeutic treatment.

If the handle unit 4X of the first model type and the transducer unit 2X of the first model type are correctly selected, as shown in FIG. 38A, the handle unit 4X of the first model type and the transducer unit 2X of the first model type are correctly coupled to the normal assembly position and are combined.

Similarly, if the handle unit 4Y of the second model type and the transducer unit 2Y of the second model type are correctly selected, as shown in FIG. 38B, the handle unit 4Y of the second model type and the transducer unit 2Y of the second model type are correctly coupled to the normal assembly position and are combined.

However, as shown in FIG. 39A, if the handle unit 4X of the first model type and the transducer unit 2Y of the second model type are combined, the hold cylinder 48X of the first model type and the C ring attachment 140Y of the second model type abut on each other at an interference part 142a. At this time, as indicated by a two-dot-and-dash line in FIG. 40A, the interference part 142a of the end face of the C ring attachment 140Y of the second model type abuts on the end face of the hold cylinder 48X of the first model type. Consequently, a further assembly operation beyond the abutment position is prevented, and the transducer unit 2Y of the second model type and the handle unit 4X of the first model type cannot be engaged to the normal assembly position (proper position).

In the case where the handle unit 4Y of the second model type and the transducer unit 2X of the first model type are combined, as shown in FIG. 39B, the hold cylinder 48Y of the second model type and the C ring attachment 140X of the first model type abut on each other at an interference part 142b. At this time, as indicated by a two-dot-and-dash line in FIG. 40B, the interference part 142b of the end face of the C ring attachment 140X of the first model type abuts on the end face of the hold cylinder 48Y of the second model type. Consequently, a further assembly operation beyond the abutment position is prevented, and the transducer unit 2X of the first model type and the handle unit 4Y of the second model type cannot be engaged to the normal assembly position (proper position).

The following advantageous effects can be obtained with the above-described structure. The handle unit 4 and transducer unit 2 of the present embodiment can be engaged in the proper position only when the handle unit 4 and transducer unit 2 are properly combined. At this time, the first to third contact-point members 131, 132 and 133 of the electrode section of the transducer unit 2, which are to be rendered electrically conductive to the handle unit 4, are surely put in contact with the handle unit 4.

Furthermore, the first to third contact-point members 131, 132 and 133 of the electrode section of the transducer unit 2 are formed to have circular cylindrical outer surfaces. Thus, in the engaged state at the proper position, the electrical conduction between the handle unit 4 and transducer unit 2 can always be ensured even if the transducer unit 2 rotates relative to the handle unit 4.

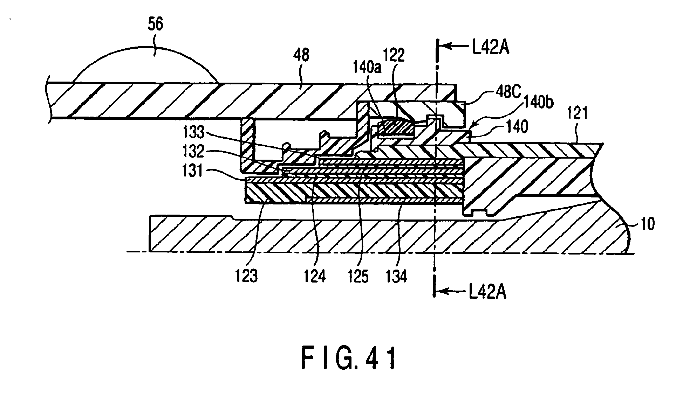

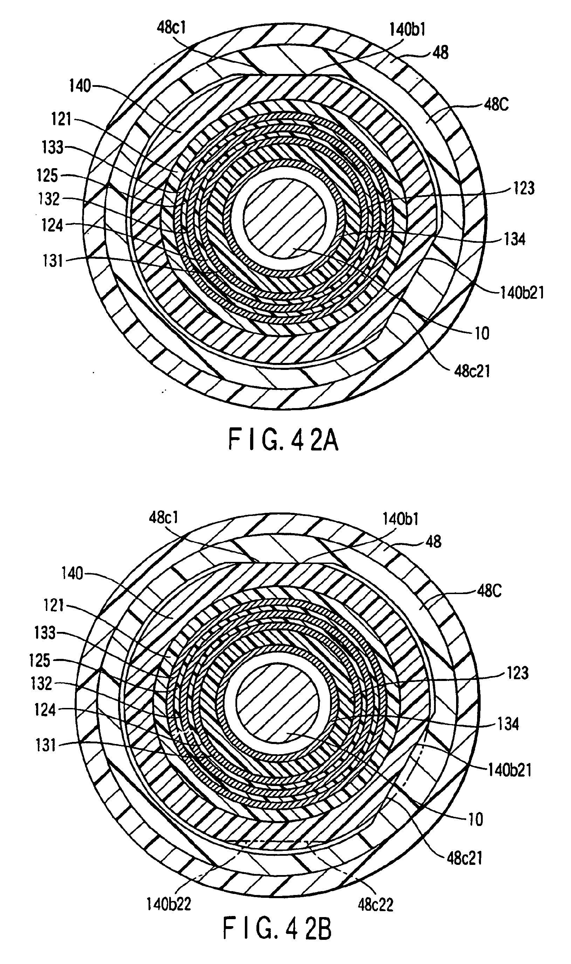

FIG. 41 and FIGS. 42A and 42B show a second embodiment of the present invention. In the present embodiment, a C ring mounting groove 140a having an annular shape and an erroneous combination prevention section (different-type assembly prevention section) 140b having an irregular shape are provided on the outer peripheral surface of the C ring attachment 140 which holds the C ring 122 of the transducer unit 2.

The irregular-shaped erroneous combination prevention section 140b, for example, in the case of the first model type, as shown in FIG. 42A, is provided with flat surfaces 140b1 and 140b21 which are formed by flattening two surface portions of the outer peripheral surface of the C ring attachment 140. The inner peripheral surface of the hold cylinder 48c of the first model type, which is normally engaged therewith, is provided with two flat surfaces 48c1 and 48c21 at parts corresponding to the flat surfaces 140b1 and 140b21 of the C ring attachment 140. The two flat surfaces 48c1 and 48c21 are disposed, for example, in an inclined surface state.

In the first model type of the present embodiment, in the case where the handle unit 4 and transducer unit 2 are in the proper combination state, the flat surfaces 140b1 and 140b21 of the C ring attachment 140 are engaged with the flat surfaces 48c1 and 48c21 of the hold cylinder 48c, and thereby engagement in the proper position can be achieved. In this case, electrical conduction between the handle unit 4 and transducer unit 2 can always be ensured even if the transducer unit 2 rotates relative to the handle unit 4.

In the second model type, a flat surface 140b22 is provided by displacing the position of the flat surface 140b21 which is one of the two flat surfaces 140b1 and 140b21 on the outer peripheral surface of the C ring attachment 140. The two flat surfaces 140b1 and 140b22 are disposed, for example, in parallel. The inner peripheral surface of the hold cylinder 48c of the second model type, which is normally engaged therewith, is similarly provided with two flat surfaces 48c1 and 48c22 at parts corresponding to the flat surfaces 140b1 and 104b22 of the C ring attachment 140.

In the second model type of the present embodiment, too, in the case where the handle unit 4 and transducer unit 2 are in the proper combination state, the flat surfaces 140b1 and 140b22 of the C ring attachment 140 are engaged with the flat surfaces 48c1 and 48c22 of the hold cylinder 48c, as shown in FIG. 42A, and thereby engagement in the proper position can be achieved. In this case, electrical conduction between the handle unit 4 and transducer unit 2 can always be ensured even if the transducer unit 2 rotates relative to the handle unit 4.

In the case of erroneous combination between the handle unit 4 and transducer unit 2, the operation is as follows. Specifically, in the case where the handle unit 4 of the first model type and the transducer unit 2 of the second model type are combined, partial interference occurs at engagement parts between the two flat surfaces 140b1 and 140b21 on the outer peripheral surface of the C ring attachment 140 and the flat surfaces 48c1 and 48c22 of the hold cylinder 48, as indicated by imaginary lines in FIG. 42B. Thereby, a further assembly operation beyond the position of interference is prevented, and the handle unit 4 of the first model type and the transducer unit 2 of the second model type cannot be engaged to the normal assembly position (proper position).

The same applies to the case of erroneous combination between the transducer unit 2 of the first model type and the handle unit 4 of the second model type.

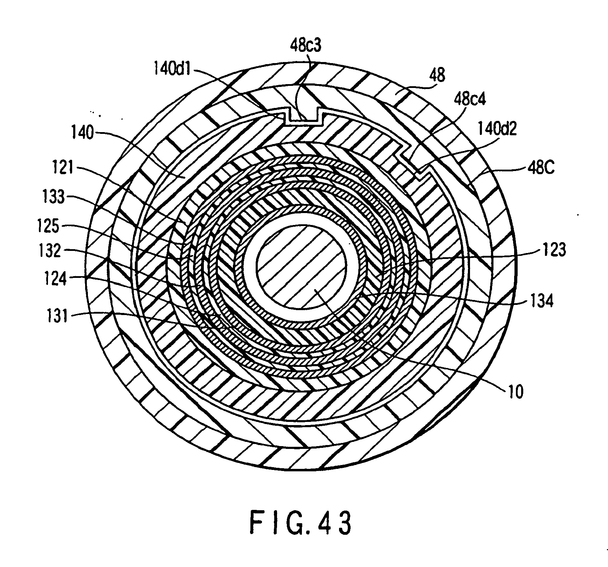

FIG. 43 shows a modification of the second embodiment. In this modification, two key grooves 140d1 and 140d2 are provided in the outer peripheral surface of the C ring attachment 140. Projection portions 48c3 and 48c4, which are engaged with the key grooves 140d1 and 140d2, are provided on the inner peripheral surface of the hold cylinder 48c. By altering the positions of the two key grooves 140d1 and 140d2 in accordance with model types, the combination between the handle unit 4 and transducer unit 2 can be restricted to a correct one.

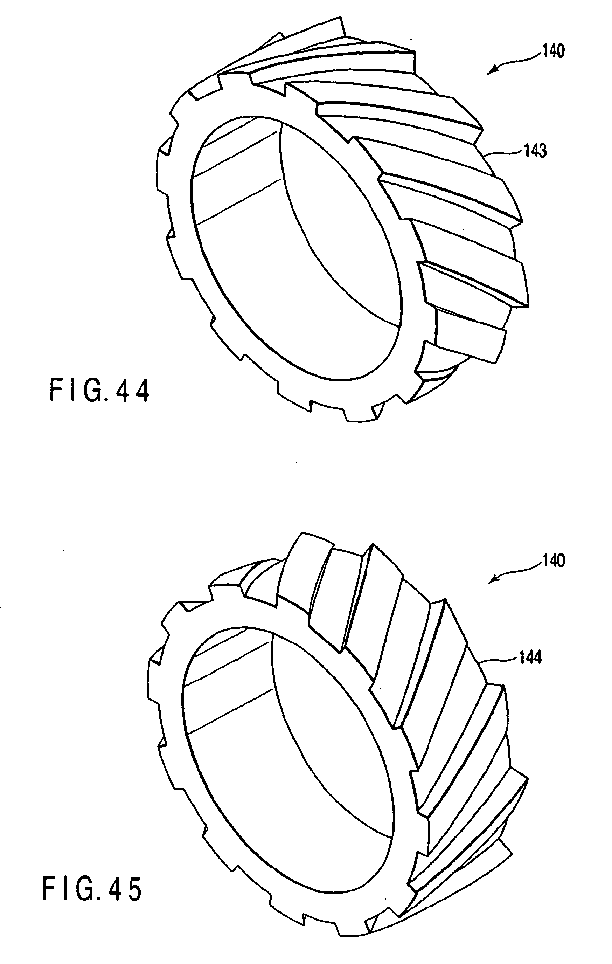

FIG. 44 and FIG. 45 show a third embodiment of the present invention. In the present embodiment, as shown in FIG. 44, a right-hand helical groove 143 is provided on the outer peripheral surface of the C ring attachment 140 of the first model type. A right-hand helical groove, which is meshed with the right-hand helical groove 143, is provided on the inner peripheral surface of the hold cylinder 48c of the first model type.

In addition, as shown in FIG. 45, a left-hand helical groove 144 is provided on the outer peripheral surface of the C ring attachment 140 of the second model type. A left-hand helical groove, which is meshed with the left-hand helical groove 144, is provided on the inner peripheral surface of the hold cylinder 48c of the second model type.

Thereby, a device with the right-hand helical groove 143 and a device with the left-hand helical groove 144 are prevented from being erroneously combined.

Besides, by altering the width (interval) or pitch of helical grooves in accordance with model types, erroneous combination of different model types can be prevented.

The present invention is not limited to the above-described embodiments. For example, the above-described embodiments are directed to bipolar-type high-frequency electric operating apparatuses, but the invention is applicable to monopolar-type high-frequency electric operating apparatuses. Needless to say, other various modifications may be made without departing from the scope of the invention.

## Claims

1. An ultrasonic operating apparatus comprising:
an ultrasonic transducer (6) which generates ultrasonic vibration;
a probe unit (3) which is coupled to the ultrasonic transducer (6) and transmits the ultrasonic vibration from the ultrasonic transducer (6);
an ultrasonic transducer unit (2) having a casing section (7) which stores the ultrasonic transducer (6);
an electric cable (9) which is provided at a proximal end portion of the ultrasonic transducer unit (2);
a handle unit (4) in which the probe unit (3) is inserted and which includes a coupling end portion for coupling to a distal end portion of the ultrasonic transducer unit (2); and
a coupling section which couples an outer periphery of the distal end portion of the ultrasonic transducer unit (2) and a proximal end portion of the handle unit (4),
**characterized in that**
the coupling section includes a different-type assembly prevention section (140b) which prevents assembly between the ultrasonic transducer unit (2) and a handle unit (4) when they are of different types, wherein
the different-type assembly prevention section (140b) includes an irregular-shaped section in which a shape or a dimension of the coupling section between the distal end portion of the ultrasonic transducer unit (2) and the proximal end portion of the handle unit (4) is varied in accordance with a plurality of model types.

2. The ultrasonic operating apparatus according to claim 1, **characterized by** further comprising a first high-frequency electric path (13) through which a high-frequency electric current is transmitted between the electric cable (9) and the probe unit (3).

3. The ultrasonic operating apparatus according to claim 2, **characterized by** further comprising a second high-frequency electric path (97) through which a high-frequency electric current is transmitted between the electric cable (9) and a sheath unit (5) which contains the probe unit (3).

4. The ultrasonic operating apparatus according to claim 1, **characterized in that** the different-type assembly prevention section (140b) includes key grooves (140d1, 140d2), which is disposed at different positions in accordance with a plurality of model types, and engaging portions (48c3, 48c4), which is engaged with the key grooves (140d1, 140d2), at the coupling section between the distal end portion of the ultrasonic transducer unit (2) and the proximal end portion of the handle unit (4).

5. The ultrasonic operating apparatus according to claim 1, **characterized in that** the ultrasonic transducer unit (2) is rotatable, relative to the handle unit (4), about an axis of the handle unit (4) in a state of assembly in a proper position.

## Patentansprüche

1. Ultraschalloperationsvorrichtung umfassend:
einen Ultraschallwandler (6) der eine Ultraschallschwingung erzeugt;
eine Sondeneinheit (3) die mit dem Ultraschallwandler (6) verbunden ist und die Ultraschallschwingung des Ultraschallwandlers (6) überträgt;
eine Ultraschallwandlereinheit (2) mit einem Gehäuseabschnitt (7) der den Ultraschallwandler (6) aufnimmt;
ein Elektrokabel (9) das an einem proximalen Endbereich der Ultraschallwandlereinheit (2) vorgesehen ist;
eine Halteeinheit (4) in die die Sondeneinheit (3) eingeführt ist und die einen Verbindungsendbereich zum Verbinden mit einem distalen Endbereich der Ultraschallwandlereinheit aufweist; und
einen Verbindungsabschnitt der einen äußeren Rand des distalen Endbereichs der Ultraschallwandlereinheit (2) und einen proximalen Endbereich der Halteeinheit (4) verbindet,
**dadurch gekennzeichnet, dass**
der Verbindungsabschnitt einen Abschnitt zum Verhindern des Zusammenbaus unterschiedlicher Typen (140b) aufweist, der einen Zusammenbau der Ultraschallwandlereinheit (2) und einer Halteeinheit (4) verhindert, wenn diese unterschiedlichen Typen zuzuordnen sind, wobei
der Abschnitt zum Verhindern des Zusammenbaus unterschiedlicher Typen (140b) einen unregelmäßig geformten Abschnitt aufweist, bei dem eine Form oder eine Abmessung des Verbindungsabschnitts zwischen dem distalen Endbereich des Ultraschallwandlers (2) und des proximalen Endbereichs der Halteeinheit (4) gemäß einer Vielzahl von Modeltypen variiert wird.

2. Ultraschalloperationsvorrichtung nach Anspruch 1 **dadurch gekennzeichnet, dass** sie ferner einen ersten elektrischen Hochfrequenzpfad (13) umfasst, durch den ein hochfrequenter elektrischer Strom zwischen dem Elektrokabel (9) und der Sondeneinheit (3) übertragen wird.

3. Ultraschalloperationsvorrichtung nach Anspruch 2 **dadurch gekennzeichnet, dass** sie ferner einen zweiten elektrischen Hochfrequenzpfad (97) umfasst, durch den ein hochfrequenter elektrischer Strom zwischen dem Elektrokabel (9) und einer Umhülleinheit (5), die die Sondeneinheit (3) beinhaltet, übertragen wird.

4. Ultraschalloperationsvorrichtung nach Anspruch 1, **dadurch gekennzeichnet, dass** der Abschnitt zum Verhindern des Zusammenbaus unterschiedlicher Typen (140b) an dem Verbindungsabschnitt zwischen dem distalen Endbereich des Ultraschallwandlers (2) und dem proximalen Endbereich der Halteeinheit (4) Passnuten (140d1, 140d2), die an unterschiedlichen Positionen gemäß einer Vielzahl von Modeltypen angeordnet sind, und Eingreifbereiche (48c3, 48c4), die in die Passnuten (140d1, 140d2) eingreifen, aufweist.

5. Ultraschalloperationsvorrichtung nach Anspruch 1, **dadurch gekennzeichnet, dass** die Ultraschallwandlereinheit (2) im zusammengebauten Zustand in eine geeignete Position, relativ zur Halteeinheit (4), um eine Achse der Halteeinheit (4) gedreht werden kann.

## Revendications

1. Appareil à fonctionnement ultrasonore comprenant :
un transducteur ultrasonore (6) qui génère une vibration ultrasonore ;
une unité de sonde (3) qui est couplée au transducteur ultrasonore (6) et transmet la vibration ultrasonore provenant du transducteur ultrasonore (6) ;
une unité de transducteur ultrasonore (2) ayant une section de boîtier (7) qui reçoit le transducteur ultrasonore (6) ;
un câble électrique (9) qui est prévu au niveau d'une partie d'extrémité proximale de l'unité de transducteur ultrasonore (2) ;
une unité de poignée (4) dans laquelle l'unité de sonde (3) est insérée et qui inclut une partie d'extrémité de couplage pour un couplage à une partie d'extrémité distale de l'unité de transducteur ultrasonore (2) ; et
une section de couplage qui couple une périphérie extérieure de la partie d'extrémité distale de l'unité de transducteur ultrasonore (2) et une partie d'extrémité proximale de l'unité de poignée (4),
**caractérisé en ce que**
la section de couplage inclut une section d'empêchement d'assemblage de types différents (140b) qui empêche un assemblage entre l'unité de transducteur ultrasonore (2) et une unité de poignée (4) lorsqu'elles sont de types différents, dans lequel
la section d'empêchement d'assemblage de types différents (140b) inclut une section de forme irrégulière dans laquelle une forme ou une dimension de la section de couplage entre la partie d'extrémité distale de l'unité de transducteur ultrasonore (2) et la partie d'extrémité proximale de l'unité de poignée (4) est fait varier conformément à une pluralité de types de modèles.

2. Appareil à fonctionnement ultrasonore selon la revendication 1, **caractérisé en ce que** comprenant en outre un premier passage électrique haute fréquence (13) à travers lequel un courant électrique haute fréquence est transmis entre le câble électrique (9) et l'unité de sonde (3).

3. Appareil à fonctionnement ultrasonore selon la revendication 2, **caractérisé en ce que** comprenant en outre un deuxième passage électrique haute fréquence (97) à travers lequel un courant électrique haute fréquence est transmis entre le câble électrique (9) et une unité de gaine (5) qui contient l'unité de sonde (3).

4. Appareil à fonctionnement ultrasonore selon la revendication 1, **caractérisé en ce que** la section d'empêchement d'assemblage de types différents (140b) inclut des rainures de clavetage (140d1, 140d2) qui sont disposées en des positions différentes conformément à une pluralité de types de modèles, et des parties d'engagement (48c3, 48c4) qui sont engagées avec les rainures de clavetage (140d1, 140d2), au niveau de la section de couplage entre la partie d'extrémité distale de l'unité de transducteur ultrasonore (2) et la partie d'extrémité proximale de l'unité de poignée (4).

5. Appareil à fonctionnement ultrasonore selon la revendication 1, **caractérisé en ce que** l'unité de transducteur ultrasonore (2) est rotative, par rapport à l'unité de poignée (4), autour d'un axe de l'unité de poignée (4) dans un état d'assemblage dans une position correcte.
